# EUROPEAN PATENT SPECIFICATION

(11) **EP 2 847 807 B1**
(45) Date of publication and mention of the grant of the patent: **27.11.2019**
(21) Application number: 13718499.0
(22) Date of filing: 12.04.2013
(51) Int. Cl.: H01L 51/46

(54) **FORMULATION COMPRISING IONIC ORGANIC COMPOUNDS FOR USE IN ELECTRON TRANSPORT LAYERS**
FORMULIERUNG MIT IONISCHEN ORGANISCHEN VERBINDUNGEN ZUR VERWENDUNG IN ELEKTRONENTRANSPORTSCHICHTEN
FORMULATION COMPRENANT DES COMPOSÉS ORGANIQUES IONIQUES POUR UTILISATION DANS DES COUCHES DE TRANSPORT D'ÉLECTRONS

(30) Priority: 10.05.2012 EP 12003723
(43) Date of publication of application: 18.03.2015
(73) Proprietor: Merck Patent GmbH, 64293 Darmstadt (DE)
(72) Inventor: BERNY, Stephane, Southampton SO15 5 BQ (GB); TOPLEY, Amy, Southampton SO18 3NB (GB); TIWANA, Priti, Winchester SO23 9RJ (GB); CULL, Toby, Romsey SO51 5PH (GB); CARRASCO-OROZCO, Miguel, Winchester SO21 3BU (GB); BLOUIN, Nicolas, Southampton SO16 7DE (GB)
(86) International application number: PCT/EP2013/001078
(87) International publication number: WO 2013/167224

(56) References cited:
- EP-A1- 2 405 529
- EP-A2- 1 107 333
- WO-A1-2011/132697
- US-A1- 2007 215 879
- US-A1- 2010 145 062
- KATRIZKY A R ET AL: "BRIDGED CYANINE DYES. PART 3 not 1 3/4 . ZWITTERIONIC BIS-4-PYRIDYL AND CATIONIC BIS-DIMETHYLANILINO DERIVATIVES", JOURNAL OF HETEROCYCLIC CHEMISTRY, WILEY-BLACKWELL PUBLISHING, INC, US, vol. 25, 1 January 1988 (1988-01-01), pages 1321-1325, XP000980404, ISSN: 0022-152X, DOI: 10.1002/JHET.5570250510

## Description

### Technical Field

The invention relates to devices selected from organic photovoltaic devices (OPV), organic solar cells, photodetectors or a component thereof, or an assembly comprising it, which comprises a layer comprising ionic organic compounds for use in electron transport layers or electron collecting layers of organic electronic (OE) devices, more specifically in organic photovoltaic (OPV) devices, to electron transport layers comprising or being made through the use of such formulations, and to OE and OPV devices comprising such formulations or electron transport layers.

### Background

Interest for interfacial layers, also called buffer layers, has considerably risen over the last few years in electronic devices such as organic Light-Emitting Diodes (OLEDs), Organic Photovoltaics (OPVs), and Field-Effect Transistors (OFETs). Once integrated into solar cells, these interfacial materials, which are by definition enclosed between the photoactive film and one of the electrodes, lead to an improvement in the performance and lifetime of the devices.

The process of light conversion into electricity in organic solar cell can be explained by a sequence of steps of different complexity; these are:
1) light absorption and exciton creation within the active layers, usually a BHJ (bulk heterojunction) consisting of a blend of Electron-Donating and /Electron-Accepting (D/A) organic compounds,
2) exciton migration to D/A physical interfaces followed by their dissociation into free charge carriers,
3) free electrons (holes) diffusion within Acceptors (Donors) domains,
4) extraction of the electrons (holes) from the BHJ to the cathode (anode).

The last step may be aided by the presence of an interfacial layer which comprises a material having either electron transport or hole transport properties, and which can thus act as electron transport layer (ETL) or hole transport layer (HTL).

Interfacial layers are thus not involved in the charge carrier generation process itself, and can be considered as not active part of the organic photovoltaic (OPV) device. Such interfacial layers can act:
1) to change the electron (hole) energy barrier at the cathode (anode) enhancing their extraction,
2) to provide selective energy barriers helping to decrease recombination between carriers of different signs,
3) to change the polarity of devices,
4) as a mechanical/chemical protective layer or seed layer for the BHJ,
5) to redistribute the incident photon flux throughout the BHJ acting as optical spacers.

Key consideration when choosing an interfacial material to improve device performance are their opto-electronic properties and their processability. Requirements for interface materials include, for example, a suitable thickness and roughness, a good coverage and chemical interaction with underneath layers or with substrates, a high microstructural uniformity, a high light transmission, selective energy levels, and a high charge carrier mobility.

Various materials have been described in the literature for use as interfacial layers, including organic and inorganic materials. One possible classification, depending on the nature of the materials, comprises three main types of interfacial layers:
1) conducting layers,
2) semiconducting layers,
3) dipole layers.

Regarding the first type, suitable materials for conducting interfacial layers include metals such as Ag, Au, Al, Ca, Mg, Pt, Ba, or combinations of metals, furthermore transparent conductive oxides (TCOs) such as ITO, IrOₓ, AlOₓ, CuOₓ, NiOₓ, HfOₓ, B:ZnO, Ga:ZnO, AI:ZnO or a combination of materials and alloys thereof. Other suitable materials include carbon derivatives such as carbon nanotubes (CNTs). Still further suitable materials include combinations of charged polymers, such as PEDOT:PSS, PANI:PSS, PPy:PSS for example.

Regarding the second type, suitable materials for semiconducting interfacial layers include inorganic oxides, such as TiOₓ, BaTiOₓ, ZnO, SnGaZnO, InGaZnO, WOₓ, MoOₓ, VOₓ, ZrOₓ. and organic small molecules like CBP, PTCDI, NPD, TCTA, TPDSi₂, or C₆₀ derivatives for example, furthermore polymers such as TFB, PFN, PVK, or a combination of such materials.

Regarding the third type, suitable materials for dipole interfacial layers comprise neutral small-molecular weight organic compounds made of electron-donating and electron-accepting moities facing to each other, creating the intrinsic electric dipole, and being chemically linked to the underneath layer with a head group.

In organic solar cells, the choice of the interfacial layer determines the collection efficiency of the free charge carriers, and thus drastically impacts on device performance and electrical stability.

However, existing conducting, semiconducting, or dipole layers being used as Electron-Transporting Layers generally match only few of the specific physical properties required to increase the overall photovoltaic parameters of the devices (V_{OC}, J_{SC}, and FF). Besides, their chemical interactions with underneath or top layers such as BHJs, substrates, electrodes or with the environment (for example oxygen or humidity) can be a problem for the stability of devices. Finally, in moving from research to mass production, processes and solvents must be carefully considered to take into consideration their environmental and economical implications. Inorganic conducting interfacial layers, for example, enable highly efficient and stable devices making use of the so-called inverted architecture, however, their processability still needs to be improved in order to be compatible with plastic substrates.

OPV devices were initially developed in the so-called regular structure. In this structure a transparent electrode, usually ITO, acts as anode on top of which a hole transport layer is deposited, which is commonly made of PEDOT:PSS. A photoactive layer is deposited on top of said hole transport layer. An interfacial layer acting as electron transport or electronic collecting layer is deposited on top of the photoactive layer, and a cathode is deposited on top of the interfacial layer.

The most commonly used interfacial layer for the transport or collection of electrons is a conducting layer of Calcium. Generally, this layer is 5nm to 50nm thick and enclosed between the photosensitive BHJ and the cathode made of Aluminum. This material, chosen because of its very low work function (W_{f}), enhances the electron-ohmic contact at the cathode and mainly improves PCE within V_{OC}. However, the Calcium layer can decompose with time and diffuse into the BHJ. This counter-productive effect dramatically decreases devices performance and lifetime. In addition to the stability issues, Calcium deposition constitutes a considerable bottleneck for manufacturing processes aiming at high-through put and low-cost OPV devices. For example, Calcium cannot be printed by means of liquid-phase formulations, but requires an expensive and time consuming vacuum-thermal evaporation process, typically in a separate chamber from the production line. Besides, this fabrication process is harsh, meaning that hot evaporated metal atoms can chemically degrade the first few nanometers of the soft organic materials used into BHJs. Although other alternative conducting layers made of metals as Barium or Magnesium, for example, have been tried as alternatives, the resulting devices could not reach the same efficiency as those made with Calcium, because these alternative interfacial layer materials do not suit the physical properties to the same extent as Calcium. Furthermore, these conducting layers still suffer from stability and cost issues.

In prior art it has also been suggested to use semiconducting layers as ETLs in OPV devices with regular architecture, in replacement of conducting Calcium layers. The most common materials are based on metal oxides such as TiOₓ. However, several problems are encountered with their use. For example, their fabrication process can require an annealing step at temperatures from 150°C to 300°C in order to decompose organo-metallic sources (usually based on acetates derivatives) leading to oxides. Such high temperatures can lead to degradation of the morphology of the underlying organic BHJ, as well as of the back substrate, especially if the back substrate is made of organic polymers like PET or PEN. It is also possible that the heating step has to be combined with another processing step of UV treatment, which is expensive and detrimental for the performances of OPV devices as well.

Dipole layers have been developed in order to overcome some of these problems. However, when such dipole layers are made of mineral salts such as LiF, Li₂CO3, Na₂CO₃, K₂CO₃, Cs₂CO₃, CsF, Cs(acac), vacuum thermal evaporation proccesses are usually required. Another alternative are dipole layers made of neutral self-assembled molecules, however, their full integration is problematic as they generally require the use of toxic solvents such as methanol. Besides, these compounds need to be chemically grafted to be fully efficient, which requires several time-consuming steps and can lead to incomplete coverage of the underlying layer.

Dipole layers comprising conjugated polymer electrolytes (CPEs) have also been shown to efficiently act as ETLs in regular OPV devices replacing Calcium. Because the cores of these organic compounds are made of semiconducting moieties (for example fluorene, thiophene, BTZ or carbazole units), they can absorb a small part of the incident light. This could lead to current generation by means of a charge transfer to the acceptor material within BHJs. Besides, the desired physical properties of these compounds strongly depend on the orientation of their dipole. However, due to the fact that the dipoles are located at the end of long alkyl chains, their reorientation can easily occur and it can also happen that the counter-ion migrates; both situations lead to a drop in the performances of the devices. The synthesis and purification steps of CPEs raises also several difficulties compared to those of small molecules. Strong dewetting effects have also been observed on layers made of CPEs, meaning that the physical properties of the resulting layer are not homogeneous, which can potentially act to reduce devices performances. The same limitations in their physical properties, processing were observed for quinacridone derivatives incorporating pendant groups as ETLs in regular OPV devices.

Thus there is still a need for materials that are suitable for use in interfacial layers, like ETLs, of OPV devices, which do not have the above-mentioned drawbacks, and have improved properties. In particular it is desired to have interfacial layer and ETL materials which are easy to synthesize, especially by methods suitable for mass production, show good structural organization and film-forming properties, exhibit good electronic properties, especially a high charge carrier mobility, a good processibility, a high solubility in organic solvents, and high stability in air. Especially for use in OPV cells, there is a need for ETL materials which enable efficient charge transport from the photoactive layer to the cathode and lead to higher cell efficiencies, compared to materials from prior art.

It was an aim of the present invention to provide materials for use in ETLs of OE devices, especially in OPV devices having regular or inverted structures, which do not show the above-mentioned drawbacks and show the desired advantageous properties as mentioned above. Another aim of the invention was to extend the pool of ETL materials available to the expert. Other aims of the present invention are immediately evident to the expert from the following detailed description.

The inventors of the present invention have found that one or more of the above aims can be achieved by providing materials and formulations as disclosed and claimed hereinafter. These formulations comprise an organic salt in solution, the salt comprising at least a first ionic entity which is a small molecule and comprises a charged organic core having a single charge or a plurality of charges, the salt further comprising a second counterionic entity of opposite sign than the first entity, which is organic, inorganic or organo-metallic, the solution further comprising polar solvents such as water, alcohols or polar non-alcoholic organic solvents.

The present invention more specifically relates to the replacement of Calcium ETLs in OPV devices by using a layer processed from a formulation comprising solvents like water, alcohol or other polar organic solvents, or combinations thereof, and an organic salt as defined hereinafter. The materials in accordance with the present invention are characterized by the nature of the salt, which is based on two individual ionic compounds, both being small molecular weight entities and having net opposite signs. OPV devices comprising an ETL prepared from such materials show V_{OC}, FF, and J_{SC} comparable or improved versus OPV devices with a Calcium ETL, and much higher performance compared to OPV devices having only a cathode of Aluminium without an ETL.

Organic charged compounds have been disclosed in prior art for use as dipole layers. T.V. Pho et al., Adv. Funct. Mater. 2011, 21, 4338-4341 disclose negatively charged small molecules based on quinacridone for use in ETLs of OLEDs and OPV devices. These molecules consist of a neutral quinacridone core and one or more negatively charged sulfonate groups attached thereto via hexylene chains. The ETL further comprises Sodium counterions, leading to a globally neutral mixture. An ETL layer is deposited from a methanol solution of these compounds between a BHJ of {PCDTBT:PC₇₁BM} or {Si-PCDTBT:PC₇₁BM} and an Aluminium cathode in a regular OPV device architecture improves the electron collection efficiency as observed by an increase in Fill Factor (FF) values.

However, the quinacridone derivatives and formulations as disclosed in the above document possess several drawbacks. Thus, due to the quinacridone core the optical bandgap is rather small (approx. 2eV), which can reduce the incident light coming into inverted OPV devices, if used as ETLs. Besides, they do not possess any hole-blocking properties. Also, the use of methanol solutions does not fit industry standards because methanol is a toxic solvent with a very high boiling point. Moreover, the quinacridone derivatives are processed by spin-coating, which is not compatible with a roll-to-roll production line and leads to a waste of material. Finally, it has been demonstrated that the increase in PCE of devices incorporating quinacridone-based ETLs in regular cells is due to an increase in FF only. Such ETLs thus do not increase the Voc as compared to control devices without buffer layer. This suggests that the dipolar character of these ETLs does not play any role in device performance.

Charged macromolecules, like cationic or anionic polymers in conjunction with counterions, have also been disclosed in prior art for use in ETLs of OLEDs (see WO 2006/029226 A1, US 2010/0096656 A1, US 2009/0230362 A1, WO 2007/126929 A2) and of OPV devices (see WO 2005/064702 A1). The core of these polymers is neutral and the charges are located on the termination of peripherical alkyl chains and counterions.

Zwitterionic compounds have also been disclosed in prior art for use in ETLs. US 2010/0145062 A1 discloses zwitterionic tetrakis(1-imidazolyl) borates comprising a central, negatively charged boron atom, positively charged imidazolium groups and negative counterions, for use in the ETL of a polymer light emitting diode (PLED). Sun et al, ACS Applied Materials & Interfaces, published in the internet in April 2012, discloses a layer of a zwitterionic compound, comprising a cationic moiety which is an N-heterocyclic, ammonium or phosphonium moiety, and an anionic sulfonyl moiety attached thereto via an alkyl chain, which is deposited on the cathode of a polymer solar cell (PSC) to increase its work function. WO 2005/055286 A2 discloses a zwitterionic compound comprising a cationic moiety, which is a heterocyclic, ammonium, guanidinium or phosphonium moiety, and an anionic sulfonyl moiety attached thereto via an alkylene spacer, and its use in the charge carrier transport layer of Dye Sensitized Solar Cells (DSSCs). Since the zwitterionic compounds do not have a net charge, they do not need a counterion and are thus not salts.

US 2007/215879 A1 discloses an organic opto-electronic device comprising (a) a cathode comprising at least one zero-valent metal; (b) an anode; and (c) an opto-electronically active organic material between said cathode and said anode; wherein said cathode is in contact with at least one organic ammonium salt.

US 2013/0092906 A1, corresponding to WO 2011/132697 A1, discloses an organic light emitting device which has an anode and a cathode, has between the anode and the cathode a light emitting layer containing a light emitting organic compound, and has between the anode and the light emitting layer a functional layer containing an ionic liquid and an organic compound (abstract).

However, the above-mentioned documents do neither disclose nor suggest compounds or formulations and their uses as claimed hereinafter.

### Summary

The invention relates to the device of claim 1 and to the process of claim 7 for producing such device, with preferred embodiments being in the dependent claims.

### Detailed Description

As used herein, the term "polymer" will be understood to mean a molecule of high relative molecular mass, the structure of which essentially comprises the multiple repetition of units derived, actually or conceptually, from molecules of low relative molecular mass (Pure Appl. Chem., 1996, 68, 2291). The term "oligomer" will be understood to mean a molecule of intermediate relative molecular mass, the structure of which essentially comprises a small plurality of units derived, actually or conceptually, from molecules of lower relative molecular mass (Pure Appl. Chem., 1996, 68, 2291). In a preferred meaning as used herein present invention a polymer will be understood to mean a compound having > 1, i.e. at least 2 repeat units, preferably ≥ 5 repeat units, and an oligomer will be understood to mean a compound with > 1 and < 10, preferably < 5, repeat units.

As used herein, the terms "monomer", "small molecule" and "small molecular weight compound" are used interchangeably, and will be understood to mean a molecule of low relative molecular mass, as compared to a polymer or oligomer, the structure of which does not essentially comprise the multiple repetition of structural units.

As used herein, the terms "donor" or "donating" and "acceptor" or "accepting" will be understood to mean an electron donor or electron acceptor, respectively. "Electron donor" will be understood to mean a chemical entity that donates electrons to another compound or another group of atoms of a compound. "Electron acceptor" will be understood to mean a chemical entity that accepts electrons transferred to it from another compound or another group of atoms of a compound. (see also U.S. Environmental Protection Agency, 2009, Glossary of technical terms, http://www.epa.gov/oust/cat/TUMGLOSS.HTM or International Union or Pure and Applied Chemistry, Compendium of Chemical Terminology, Gold Book)_

As used herein, the term "n-type" or "n-type semiconductor" will be understood to mean an extrinsic semiconductor in which the conduction electron density is in excess of the mobile hole density, and the term "p-type" or "p-type semiconductor" will be understood to mean an extrinsic semiconductor in which mobile hole density is in excess of the conduction electron density (see also, J. Thewlis, Concise Dictionary of Physics, Pergamon Press, Oxford, 1973).

As used herein, the term "conjugated" will be understood to mean a compound (for example a polymer) that contains mainly C atoms with sp²-hybridisation (or optionally also sp-hybridisation), and wherein these C atoms may also be replaced by hetero atoms. In the simplest case this is for example a compound with alternating C-C single and double (or triple) bonds, but is also inclusive of compounds with aromatic units like for example 1,4-phenylene. The term "mainly" in this connection will be understood to mean that a compound with naturally (spontaneously) occurring defects, which may lead to interruption of the conjugation, is still regarded as a conjugated compound.

As used herein, the term "ionic liquid (IL)" means an organic salt that usually has a melting point below 373 K. Review articles on ionic liquids are, for example, R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Ubergangsmetallkatalyse" [Ionic Liquids - Novel Solutions for Transition-Metal Catalysis], Angew. Chem., 112 (2000), 3926-3945; T. Welton "Room temperature ionic liquids. Solvents for synthesis and catalysis", Chem. Rev., 92 (1999), 2071-2083 or R. Hagiwara, Ya. Ito "Room temperature ionic liquids of alkylimidazolium cations and fluoroanions", J. Fluorine Chem., 105 (2000), 221-227.

As used herein, the term "polymerisable ionic liquid (PIL)" means an ionic liquid with a polymerisable or crosslinkable functional group attached to the one of the ions, preferably the cation, via a spacer group.

As used herein, unless stated otherwise the molecular weight is given as the number average molecular weight Mₙ or weight average molecular weight Mw, which is determined by gel permeation chromatography (GPC) against polystyrene standards in eluent solvents such as tetrahydrofuran, trichloromethane (TCM, chloroform), chlorobenzene or 1, 2, 4-trichlorobenzene. Unless stated otherwise, 1,2,4-trichlorobenzene is used as solvent. The degree of polymerization, also referred to as total number of repeat units, n, will be understood to mean the number average degree of polymerization given as n = Mₙ/M_{U}, wherein Mₙ is the number average molecular weight and Mu is the molecular weight of the single repeat unit, see J. M. G. Cowie, Polymers: Chemistry & Physics of Modern Materials, Blackie, Glasgow, 1991.

The inventors of the present invention have found that the use of organic salts as described above and below in ETLs can solve many of the problems encountered in prior art when using classical ETLs in OPV devices, and thus provide significant advantages over the materials as used in prior art. Embodiments of the invention providing such advantages are discussed below:
The salt used in accordance with the present invention is made of small molecule compounds (also referred to as "small molecular weight" or "monomeric"). Consequently its synthesis requires less synthetic steps than that of the polymeric materials as used in prior art. The physical properties of the salt are less dependent on variable parameters like those of polymers, such as molecular weight, polydispersity, purification, or characterization, for example. This leads to a clear reduction in the cost of production and increases the performances reliability as compared to dipole mixtures based on conjugated polymer electrolytes. It also provides advantages over ZnO or TiOₓ nanoparticles, which suffer from size or structural variations from batch to batch.

In accordance with the present invention, the salt comprises two monomeric ionic entities which have opposite net signs, and at least one of which comprises an organic charged core.

The first entity comprises an organic charged core, which is for example an N-heterocyclic moiety, such as pyrrolidinium, thiazolium, oxazolium, triazolium or carbazolium, or comprise a sulfonium, guanidinium, phosphonium or ammonium moiety.

The second entity comprises, for example, a phosphate, borate, carboxylate or nitrate, if the first entity has a positive net sign. The second entity can also comprise a positively charged organic core, for example an N-heterocyclic moiety, such as pyrrolidinium, thiazolium, oxazolium, triazolium or carbazolium, or comprise a sulfonium, guanidinium, phosphonium or ammonium moiety, for example.

Both the first and the second entity can also possess a plurality of charges, distributed on the core and/or on peripherical charged alkyl chains. Both the viscosity and the solubility of the two entities of the salt can be tuned by changing their number of charges. For example, ionic-terminated alkyl chains, or zwitterionic chains can be added. Additionally, the size of the cores of the ionic entities as well as their chemical functionalities can be changed by simple synthetic modifications.

Preferred N-heterocyclic moieties are selected from the group consisting of the following structures 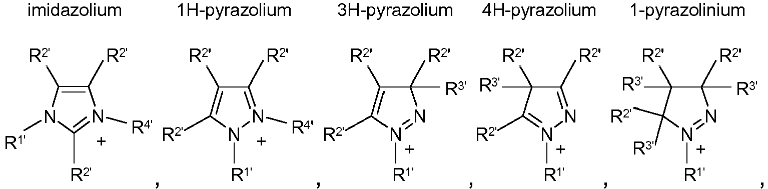 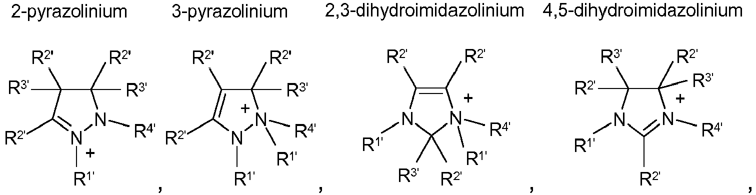 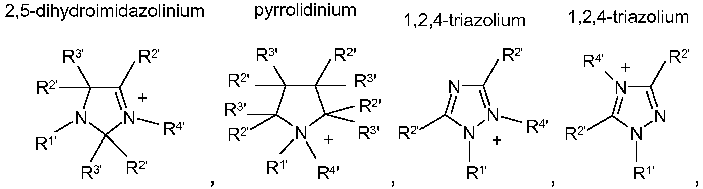 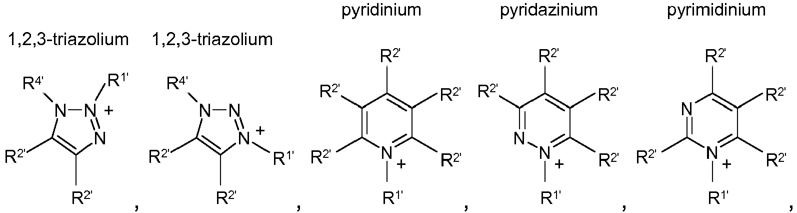 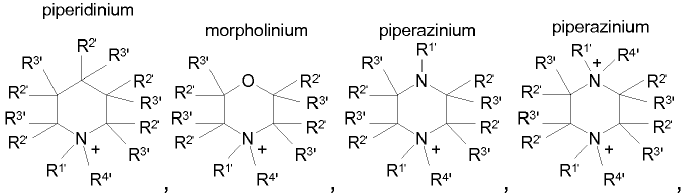 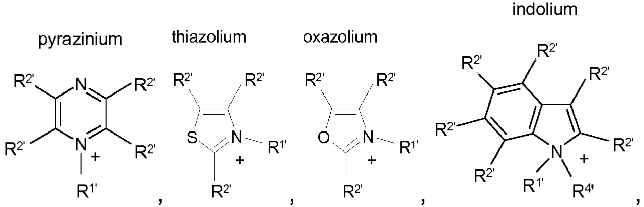 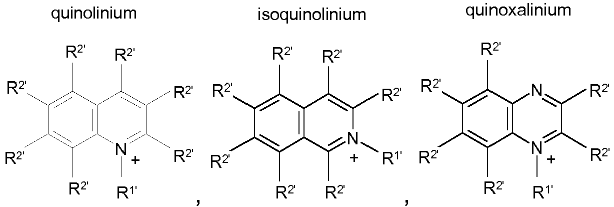 or 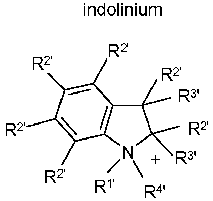 wherein the substituents R^{1'} to R^{4'} each, independently of one another, denote
- a straight-chain or branched alkyl having 1-20 C atoms, which optionally can be partially fluorinated, but not in α-position to hetero-atom, and which can also include oxygen or/and sulfur atoms in any positions in between carbon atoms, or
- saturated, partially or fully unsaturated cycloalkyl having 5-7 C atoms, which may be substituted by alkyl groups having 1-6 C atoms, and wherein the substituents R^{1'}, R^{2'}, R^{3'} and/or R^{4'} together may also form a ring system, and, in case of reactive compounds, one or more of the substituents R^{1'} to R^{4'} may also denote an alkylene spacer group Sp, preferably having from 2 to 12 C atoms, that is linked to a polymerisable or crosslinkable functional group.

The polymerisable or crosslinkable functional group is preferably an acrylate, methacrylate, fluoroacrylate, chloroacrylate, cyanoacrylate, epoxy, oxetane, vinyloxy or styryl group.

The first entity preferably comprises a moiety selected from the group consisting of dialkylpyrrolidinium, trialkylsulfonium, carbazolium, indolium, piperidinium, morpholinium, pyrimidinium, pyridazinium, pyrazinium, pyrazolium, pyrrolinium, pyrimidinium, pyrazinium, thiazolium, oxazolium, triazolium, triazinium, guanidinium, uranium, ammonium, sulfonium, phosphonium.

In another preferred embodiment the first entity comprises an organic zwitterionic compound, preferably a zwitterionic dye, very preferably selected from a cyanine dye, a merocyanine dye, a squarylium dye, or a polymethine dye, and the second entity comprises an organic or inorganic counterion.

The second entity preferably comprises an organic or inorganic moiety, very preferably a halide, borate, imide, nitrate, phosphate, sulfonate, sulfate, succinate, naphthenate, carboxylate or O-heterocyclic moiety.

In another preferred embodiment the second entity is selected from the group consisting of halides, preferably chloride, bromide or iodide, hydrogensulfate, alkylsulfates, fluoroalkyl-phosphates, hexafluorophosphate, bis(trifluoromethylsulfonyl)imide, formate, trifluoroacetate, tetrafluoroborate, oxalatoborate, tetracyanoborate, dicyanamide, tricyanomethide, thiocyanate, methanesulfonate, triflate (trifluoromethane-sulfonate), nonaflate (nonafluorobutane-sulfonate), tosylate (toluene-sulfonate) and hydrogensulfate.

Very preferably the second monomeric ionic entity is selected from the group consisting of Cl⁻, Br⁻, I⁻, [HSO₄]⁻, [CH₃SO₄]⁻, [C₂H₅SO₄]⁻, [C₄H₉SO₄]⁻, [C₆H₁₃SO₄]⁻, [C₈H₁₇SO₄]⁻, [C₅H₁₁O₂SO₄]⁻, [(C₂F₅)₃PF₃]⁻, [PF₆]⁻, [N(SO₂CF₃)₂]⁻ , [HCOO]⁻, [CF₃COO]⁻, [BF₄]⁻, [B(C₂O₄)₂]⁻, [B(CN)₄]⁻, [N(CN)₂]⁻, [C(CN)₃]⁻, [SCN]⁻, [CH₃SO3]⁻, [CF₃SO₃]⁻, [C₄F₉SO₃]⁻, [CH₃C₆H₄SO₃]⁻.

In another preferred embodiment the second entity is selected from the group consisting of chloride, bromide, iodide, tetrafluoroborate, tetracyanoborate (TCB), difluoro-dicyano borate, fluoro-tricyano borate, perfluoroalkyl-fluoro-dicyano borate, pentafluoroethyl-fluoro-dicyano borate, perfluoroalkyl-difluoro-cyano borate, pentafluoroethyl-difluoro-cyano borate, perfluoroalkyl-fluoro borate (FAB), perfluoroalkyl-alkoxy-dicyano borate, alkoxy-tricyano borate, methoxy-tricyano borate, ethoxy-tricyano borate, 2,2,2-trifluoroethoxy-tricyano borate, bis(2,2,2-trifluoroethoxy)-dicyano borate, tetraphenylborate (TPB), tetrakis(3,5-bis(trifluoromethyl)phenyl) borate (TFPB), tetrakis(4-chlorophenyl)borate, tetrakis(4-fluorophenyl) borate, tetrakis(pentafluorophenyl)borate, tetrakis(2,2,2-trifluoroethoxy) borate, bis(oxalato)borate, bis(trifluoromethylsulfonyl)imide (NTF), bis(fluorosulfonyl)imide, bis[bis(pentafluoroethyl)phosphinyl]imide (FPI), tris(trifluoromethylsulfonyl)methide, (fluoroalkyl)fluorophosphates, tris(pentafluoroethyl)trifluorophosphate (FAP), bis(pentafluoroethyl) tetrafluorophosphate, (pentafluoroethyl)pentafluorophosphate, tris(nonafluorobutyl)trifluorophosphate, bis(nonafluorobutyl)tetrafluorophosphate, (nonafluorobutyl)pentafluorophosphate, hexafluorophosphate, bis(fluoroalkyl)phosphinate, bis(pentafluoroethyl)phosphinate, bis(nonafluorobutyl) phosphinate, (fluoroalkyl)phosphonate, (pentafluoroethyl)phosphonate, (nonafluorobutyl)phosphonate, nonafluorobutane sulfonate (nonaflate) (NFS), trifluoromethanesulfonate, trifluoroacetate, methanesulfonate, butanesulfonate, butylsulfate, hexylsulfate, octylsulfate, dicyanamide, tricyanomethide, thiocyanate, hydrogensulfate, trifluoroacetate, tosylate, docusates: (bis(2-2-ethyl hexyl) sulfosuccinate (AOT), naphthenates, lauryl sulphate, alkyl benzene sulfonates (dodecyl benzene sulfonates, linear and branched), alkyl naphthalene sulfonate, alkyl aryl ether phosphates, alkyl ether phosphate, alkyl carboxylates: stearate, octoates, heptanoate, wherein preferably "alkyl" is C₁-C₂₀ alkyl, "fluoroalkyl" is fluorinated C₁-C₂₀ alkyl, "perfluoroalkyl" is C₁-C₂₀ perfluoroalkyl, and "aryl" is optionally substituted C₅-C₈-aryl, preferably benzene.

In another preferred embodiment the second entity comprises a moiety selected from the group consisting of imidazolium, dialkylimidazolium, trialkylimidazolium, dialkylpyrrolidinium, mono or dialkylpyridinium, trialkylsulfonium, carbazolium, indolium piperidinium, morpholinium, pyrimidinium, pyridazinium, pyrazinium, pyrazolium, pyrrolinium, pyrimidinium, pyrazinium, thiazolium, oxazolium, triazolium, triazinium, guanidinium, ammonium, sulfonium, phosphonium, and cations of Group 1 of the chemical table, very preferably H⁺, Na⁺ or K⁺.

In another preferred embodiment the organic salt is a ionic liquid (IL) or a polymerisable ionic liquid (PIL) comprising a, preferably only one, cation and a, preferably only one, anion. In this preferred embodiment the cation is preferably selected from the preferred organic cations as described for the first moiety above, and the anion is preferably selected from the preferred anions as described for the second moiety above.

In another preferred embodiment the IL is a PIL selected from the N-heterocyclic moieties of the structures shown above, wherein one or more of the substituents R^{1'} to R^{4'} denote an alkylene spacer group Sp, preferably having from 2 to 12 C atoms, that is linked to a polymerisable or crosslinkable functional group.

For example, the IL or PIL may comprise a cation selected from the following formulae 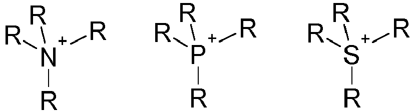 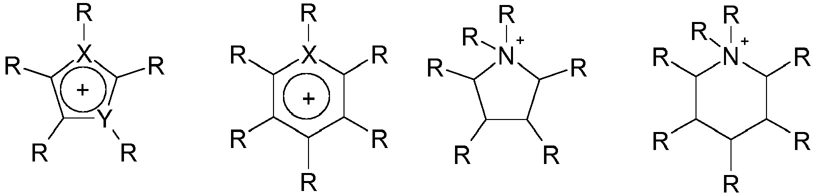 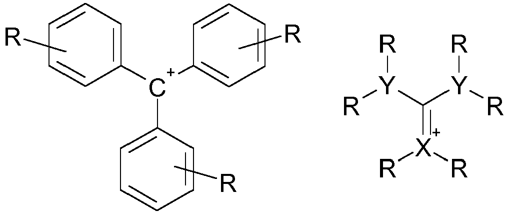 wherein R on each occurrence identically or differently denotes C₁-C₃₀ alkyl, C₁-C₃₀ alkoxy, C₁-C₃₀ thiaalkyl, C₂-C₈ alkenyl, C₂-C₈ alkynyl, C₆-C₁₂ aryl, C₆-C₁₂ cycloalkyl, C₃-C₁₂ heterocyclyl, C₃-C₁₂ heteroaryl, C₆-C₂₀ alkylaryl, C₆-C₂₀ alkylcycyl, C₃-C₁₂ alkylheteroaryl, C₃-C₂₀ alkylheterocyclyl, silane, trichlorosilane, silanol, Ph₂P(O)-, Ph₂P-, Me₂P(O)-, Me₂P-, Ph₂P(S)-, Ph₃P-N-, Me3P-N-, FSO₂CF₂-, ClSO₂(CF₂)ₙ-, HSO₃(CF₂)ₙ-, HCO₂(CF₂)ₙ-, FSO₂NHSO₂(CF₂)ₙ-, CF₃SO₂NHSO₂(CF₂)ₙ-, CₙF₂ₙ₊₁SO₂NHSO₂(CF₂)ₙ-, FSO₂(CF₂)ₙ-, ClSO₂(CF₂)ₙ-, CₙF₂ₙ₊₁SO₂NH(CF₂)ₙ-, -OH, -H, -F, -CI, -Br, -I, -CN, -NO₂, -SO₃H, C₁-C₁₂ hydroxy alkyl, or a polymerisable or crosslinkable group as defined for R^{1'} above, wherein "Ph" denotes phenyl, "Me" denotes methyl, and n is an integer from 1 to 48, preferably from 1 to 20, and wherein two adjacent substituents R can be linked to each other to form a polycyclic ring system having from 5 to 20 C atoms.

In another preferred embodiment the IL or PIL comprises an anion which selected from the group consisting of O-substituted heterocycles, halides, borates, fluoroborates, sulfonates, sulfates, phosphates, fluorophosphates, carboxylates, nitrates, very preferably from C₄F₉SO₃⁻, (C₂H₅)₂PO₄⁻ , (CH₃)₂PO₄-,CH₃SO₃⁻, C₅H₁₁O₂SO₄⁻, C₈H₁₇SO₄⁻, C₂H₅SO₄⁻, HSO₄⁻, Al₂Cl₇⁻, N(CN)₂⁻, B(CN)₄⁻, (C₂F₅)₃PF₃⁻, (FSO₂)₂N⁻, (CF₃SO₂)₂N⁻, (C₂F₅SO₂)₂N⁻, (CF₃SO₂)₃C⁻, CF₃SO₃⁻, CF₃COO⁻, AsF₆⁻, CH₃COO⁻, (CN)₂N⁻, (CN)₃C-, NO₃⁻, Cl⁻, Br⁻, I⁻, PF₆⁻, ClO₄⁻, BF₄⁻, SCN⁻, or mixtures thereof.

The present formulations further comprise a solvent or a combination of solvents. Preferably the solvents are selected from water, alcohols , non-alcoholic polar organic solvents, or combinations thereof.

Very preferred solvents include water, or an organic solvent selected from methanol, isopropanol, acetic acid, acetone, ethanol, and dimethylsulfoxide, or combinations of the aforementioned, for example a combination of water with one or more of the aforementioned organic solvents.

In case of ILs or PILs the solvent preferably comprises ethanol or isopropanol. In case of other organic salts the solvent preferably comprises a combination of water and ethanol or a combination of water and isopropanol. Such non-toxic solvents fit industries toxicity standards, which is a clear advantage compared to the overall portfolio of dipole-based formulations as described in the literature, but also as compared to semiconducting metal oxide formulations.

These polar solvents can be combined with other solvents and can also be used in different combinations and volumic ratios in order to improve both the solubility of the salt and the viscosity of the mixture at the same time. The ability of the salt to be dissolved and processed in a lot of different solvents is caused inter alia by its low molecular weight, which is a clear advantage compared to CPEs.

The organic salt is preferably dissolved in the solvent(s) at a concentration from 0.001 to 30mg/ml, very preferably from 0.1 to 2.5mg/ml.

In case the organic salt is an IL or PIL, it is preferably dissolved in the solvent(s) at a concentration from 0.0001 vol% to 10 vol%, more preferably from 0.001 vol% to 2.0 vol%.

A layer can be formed from the formulation as defined herein by a process comprising the steps of
- depositing the formulation on a substrate,
- removing the solvents for example by evaporation, preferably under heat and/or reduced pressure, and
- optionally drying the layer at a temperature above room temperature.

The present formulations may be deposited by any suitable method. Liquid coating of devices is more desirable than vacuum deposition techniques. Solution deposition methods are especially preferred. The present formulations enable the use of a number of liquid coating techniques. Preferred deposition techniques include, without limitation, dip coating, spin coating, ink jet printing, nozzle printing, letter-press printing, screen printing, gravure printing, doctor blade coating, roller printing, reverse-roller printing, offset lithography printing, dry offset lithography printing, flexographic printing, web printing, spray coating, dip coating, curtain coating, brush coating, slot dye coating or pad printing.

Ink-jet printing is particularly preferred when high resolution layers and devices needs to be prepared. Selected formulations may be applied to prefabricated device substrates by ink jet printing or microdispensing. Preferably industrial piezoelectric print heads such as but not limited to those supplied by Aprion, Hitachi-Koki, InkJet Technology, On Target Technology, Picojet, Spectra, Trident, Xaar may be used to apply the organic semiconductor layer to a substrate. Additionally semi-industrial heads such as those manufactured by Brother, Epson, Konica, Seiko Instruments Toshiba TEC or single nozzle microdispensers such as those produced by Microdrop and Microfab may be used.

In order to be applied by ink jet printing or microdispensing, the compounds or polymers should be first dissolved in a suitable solvent. Solvents must fulfil the requirements stated above and must not have any detrimental effect on the chosen print head.

The present formulations are preferably processed from solution, for example by using spin-coating or printing techniques. These printing techniques are less expensive than the vacuum thermal evaporation processes used for most of conducting layers.

The layer forming process is preferably carried out at a temperature of the substrate ranging from 10°C to 120°C, in case of ILs and PILs preferably from 10°C to 120°C in case of other organic salts preferably from 10°C to 90°C. The formulation can also be coated at Room Temperature (RT), which further decreases the cost of the production line. On the other hand this temperature range is also high enough to activate crosslinkable chemical functions in the moieties of PILs. For example processing the layer at 120°C can allow the formation of a chemically crosslinked layer made of anions (or cations) facing cations (or anions). This can lead to reinforced dipole strength with time. This activation process of crosslinkable functionalities can also be achieved or supported photochemically, for example by exposure to UV light.

As underlying substrate for example a metal oxide substrate or BHJ of an OPV device can be used. The application of the formulation of the present invention with the salt and the solvents does not change the morphology of underlying layers, or of the interfaces between the different layers of the stack by an additional annealing step either. The processing temperature can be selected to fit the requirements of plastic substrates as well.

The inventors of the present invention also observed that the enhancement in devices performance is mainly due to the presence of the salt, because they did observe any improvements in devices performances after processing only the solvents in accordance with the present invention, as contrary to studies on dipole reporting by means of solvent-washing effects.

The formulation can be processed in air or in an inert gas atmosphere, for example N₂. It was observed that when the formulation is processed in air, the device performance can be almost the same as that obtained when the formulation is processed under N₂. This is an important difference to metal oxides layers for which physical properties are sensitive to the environment on which they are processed. As a result, the fabrication costs can also be decreased and performance reliability is enhanced.

The dipole layers in accordance with the present invention do not require any further annealing or post-deposition treatment to improve their microstructures or to complete conversion if for example a pre-cursor route is needed. This is contrary to semiconducting layers based on metal oxides as used in prior art. This does also avoid damages of underlying layers, is compatible with plastic substrates requirements, and is a clear improvement compared to classical dipole-based ETLs of prior art. Without wishing to be bound to a specific theory, these advantageous results can be explained by the low global vapour pressure of solvents which naturally drive the formation of the ETL dipole layer of the present invention.

The coated layer comprising the formulation according to the present invention after solvent removal is preferably less than 50nm thick and less than 10nm rough, as measured by AFM. Its morphology is observed to be uniform. Typical dewetting issues that are usually encountered with dipole compounds can be avoided by tuning the volumic ratio of the different solvents used in the formulation. The homogeneity of the thin films also constitutes an advance compared to those of semiconducting metal oxides or classical dipole layers as used in prior art.

Another benefit of the present invention is the improved electrical performance of devices comprising the ETL in accordance with the present invention. An increase in both Voc and FF can be observed, compared to reference devices having a cathode aluminum only. This is attributed to the intrinsic physical properties of the salt.

The use of an ETL according to the present invention increases the electric field at the cathode of OPV devices, which leads to an increase in Voc. The electrical strength of dipoles is known to strongly depend on the nature of the charged entities, especially their size and orientation. A characterizing feature of the ETL material of the present invention is the presence of at least one organic charged core, which allows to extend in the electric dipole to several atoms. This effect is even more significant when the charged core possesses a plurality of charges, and when the counter-entity is a mirror compound of opposite net sign. This makes a clear difference as compared to CPEs and quinacridone derivatives of prior art, which provide shorter-distance dipoles localised on a single atom of their peripherical chains. It also makes a clear difference as compared to the compounds of prior art. for example the zwitterionic compounds as disclosed in WO 2005/055286 A1, for which steric contributions inherent to zwitterionic compounds constraint the delocalization of the electrical charge to some extent.

The increase in FF highlights both the fitted structuration of the layer and the direction of the dipole.

Another benefit when using ILs or PILs is that they are wide-bandgap materials. This intrinsic property allows their use in both regular and inverted OPV device configuration, without any optical contribution to the device working mode such as optical losses, charge transfer to acceptor or donor phase. etc. This is a clear advantage compared to the systems described in prior art for the same purpose such as CPEs, quinacridone derivatives and dyes, which are absorbing light in the visible or IR range and can thus affect OPV device performance.

The present formulations are suitable for use in the preparation of electron transport layers of electronic, electroluminescent or photoluminescent components or devices. In these devices, the present formulations are typically applied as thin layers or films.

Thus, the present invention also provides the use of the formulation in a process for preparing an ETL layer in an electronic device.

The ETL layer may be less than about 30 microns. For various electronic device applications, the thickness may be less than about 1 micron thick. The layer may be deposited, for example on a part of an electronic device, by any of the aforementioned solution coating or printing techniques.

Further preferred embodiments of the present invention are the following:
- the formulation is processed by spin-coating or other printing techniques such as slot-dye coating, inkjet printing, spray, doctor-blade on top of a substrate, preferentially by spin-coating or doctor-blading,
- the formulation is processed under an atmosphere of air, N₂ or water pressure, preferably under an N₂ atmosphere,
- the formulation is processed at a temperature from 10°C to 90°C, preferably at room temperature,
- the formulation is processed at a temperature from 10°C to 120°C, preferably at room temperature,
- the layer formed from the formulation is dried at a temperature from room temperature to 200°C, preferably from room temperature to 60°C,
- the layer formed form the formulation is crosslinked at a temperature from room temperature to 200°C and/or by UV exposure,
- the layer formed from the formulation is integrated in a device selected from the group consisting of an optical component, a magnetical component, an electrical component, an optoelectronic component, a biosensor, a photodoide, a light-emitting diode, an optoelectronic semiconductor chip, a semi-conductor thin film, a field-effect transistor, a polymeric photoswitch, an organic memory, a memristor, a transducer, a photodetector, a laser material, an optical interconnect, a light-emitting electrochemical cell, a solar cell, a phototransistor, a liquid crystal, a tunnel junction, a spin-valve device, a photovoltaic cell, preferably an organic solar cell,
- the layer formed from the formulation is capable of enhancing one or more of the transport of unipolar free charge carriers, the injection efficiency of unipolar free charge carriers, or the extraction efficiency of unipolar free charge carriers, and is capable of decreasing the recombination probability of unipolar free charge carriers in optoelectronic devices such as OPV devices, OFETs and OLEDs,
- the layer formed from the formulation acts as an electron-transporting layer (ETL) in organic electronic devices, preferably in OPV devices or organic solar cells,
- the layer formed from the formulation is provided on a substrate which is selected from an electrode, an ETL, a HTL, a BHJ, a plastic substrate, a glass substrate, a conducting layer or a semiconducting layer, preferably selected from a BHJ in an OPV device or a metal oxide semiconducting layer, conducting layer or ETLs in an inverted OPV device,
- the OPV device comprises a cathode of aluminium, gold, silver, calcium or barium,
- the ETL leads to an improved performance, preferably an increase of the V_{OC} and/or FF, of an OPV device comprising a BHJ and an aluminium electrode, compared to a control device having no ETL between the aluminium cathode and the BHJ,
- the BHJ in the OPV device comprises a polymer-fullerene mixture, and preferably comprises a fullerene selected from PC₆₀BM and PC₇₀BM, and a polymer selected from P1, P2, P3, P4 as shown in the examples.

The formulations according to the present invention can additionally comprise one or more further components or additives selected for example from surface-active compounds, lubricating agents, wetting agents, dispersing agents, hydrophobing agents, adhesive agents, flow improvers, defoaming agents, deaerators, diluents which may be reactive or non-reactive, auxiliaries, colourants, dyes or pigments, sensitizers, stabilizers, binding agents, nanoparticles or inhibitors.

The invention additionally provides an electronic device comprising an ETL according to the present invention. Especially preferred electronic devices are OPV devices, in particular bulk heterojunction (BHJ) OPV devices or inverted BHJ OPV devices.

The OPV device can for example be of any type known from the literature (see e.g. Waldauf et al., Appl. Phys. Lett., 2006, 89, 233517).

A first preferred OPV device according to the invention comprises the following layers (in the sequence from bottom to top):
- optionally a substrate,
- a high work function electrode, preferably comprising a metal oxide, like for example ITO, serving as anode,
- an optional conducting polymer layer or hole transport layer, preferably comprising an organic poymer or polymer blend, for example of PEDOT:PSS (poly(3,4-ethylenedioxythiophene): poly(styrene-sulfonate),
- a layer, also referred to as "active layer", comprising a p-type and an n-type organic semiconductor, which can exist for example as a p-type/n-type bilayer or as distinct p-type and n-type layers, or as blend or p-type and n-type semiconductor, forming a BHJ,
- an ETL layer comprising or being formed from a formulation according to the present invention,
- a low work function electrode, preferably comprising a metal like for example aluminum, serving as cathode,
wherein at least one of the electrodes, preferably the anode, is transparent to visible light.

A second preferred OPV device according to the invention is an inverted OPV device and comprises the following layers (in the sequence from bottom to top):
- optionally a substrate,
- a high work function metal or metal oxide electrode, comprising for example ITO, serving as cathode,
- an ETL layer comprising or being formed from a formulation according to the present invention,
- an active layer comprising a p-type and an n-type organic semiconductor, situated between the electrodes, which can exist for example as a p-type/n-type bilayer or as distinct p-type and n-type layers, or as blend or p-type and n-type semiconductor, forming a BHJ,
- an optional conducting polymer layer or hole transport layer, preferably comprising an organic poymer or polymer blend, for example of PEDOT:PSS,
- an electrode comprising a high work function metal like for example silver, serving as anode,
wherein at least one of the electrodes, preferably the cathode, is transparent to visible light.

The photoactive layer preferably comprises a p-type (electron donor) semiconductor and an n-type (electron acceptor) semiconductor.

The p-type semiconductor is for example a conjugated organic polymer.

In a preferred embodiment in accordance with the present invention, the p-type semiconductor is selected from conjugated polymers or copolymers that encompass one or more repeating units selected from thiophene-2,5-diyl, 3-substituted thiophene-2,5-diyl, optionally substituted thieno[2,3-b]thiophene-2,5-diyl, optionally substituted thieno[3,2-b]thiophene-2,5-diyl, selenophene-2,5-diyl, or 3-substituted selenophene-2,5-diyl.

Further preferred p-type semiconductors are selected from copolymers comprising electron acceptor and electron donor units. Preferred copolymers of this preferred embodiment are for example copolymers comprising one or more benzo[1,2-b:4,5-b']dithiophene-2,5-diyl units that are preferably 4,8-disubstituted by one or more groups R as defined above, and further comprising one or more aryl or heteroaryl units selected from Group A and Group B, preferably comprising at least one unit of Group A and at least one unit of Group B, wherein Group A consists of aryl or heteroaryl groups having electron donor properties and Group B consists of aryl or heteroaryl groups having electron acceptor properties, and preferably
Group A consists of selenophene-2,5-diyl, thiophene-2,5-diyl, thieno[3,2-b]thiophene-2,5-diyl, thieno[2,3-b]thiophene-2,5-diyl, selenopheno[3,2-b]selenophene-2,5-diyl, selenopheno[2,3-b]selenophene-2,5-diyl, selenopheno[3,2-b]thiophene-2,5-diyl, selenopheno[2,3-b]thiophene-2,5-diyl, benzo[1,2-b:4,5-b']dithiophene-2,6-diyl, 2,2-dithiophene, 2,2-diselenophene, dithieno[3,2-*b*:2',3'-*d*]silole-5,5-diyl, 4*H*-cyclopenta[2,1-*b*:3,4-*b*']dithiophene-2,6-diyl, 2,7-di-thien-2-yl-carbazole, 2,7-di-thien-2-yl-fluorene, indaceno[1,2-b:5,6-b']dithiophene-2,7-diyl, benzo[1",2":4,5;4",5":4',5']bis(silolo[3,2-b:3',2'-b']thiophene)-2,7-diyl,2,7-di-thien-2-yl-indaceno[1,2-b:5,6-b']dithiophene,2,7-di-thien-2-yl-benzo[1",2":4,5;4",5":4',5']bis(silolo[3,2-b:3',2'-b']thiophene)-2,7-diyl, and 2,7-di-thien-2-yl-phenanthro[1,10,9,8-*c,d,e,f,g*]carbazole, all of which are optionally substituted by one or more, preferably one or two groups R, and
Group B consists of benzo[2,1,3]thiadiazole-4,7-diyl, 5,6-dialkyl-benzo[2,1,3]thiadiazole-4,7-diyl, 5,6-dialkoxybenzo[2,1,3]thiadiazole-4,7-diyl, benzo[2,1,3]selenadiazole-4,7-diyl, 5,6-dialkoxy-benzo[2,1,3]selenadiazole-4,7-diyl, benzo[1,2,5]thiadiazole-4,7,diyl, benzo[1,2,5]selenadiazole-4,7,diyl, benzo[2,1,3]oxadiazole-4,7-diyl, 5,6-dialkoxybenzo[2,1,3]oxadiazole-4,7-diyl, 2H-benzotriazole-4,7-diyl, 2,3-dicyano-1,4-phenylene, 2,5-dicyano,1,4-phenylene, 2,3-difluro-1,4-phenylene, 2,5-difluoro-1,4-phenylene, 2,3,5,6-tetrafluoro-1,4-phenylene, 3,4-difluorothiophene-2,5-diyl, thieno[3,4-b]pyrazine-2,5-diyl, quinoxaline-5,8-diyl, thieno[3,4-b]thiophene-4,6-diyl, thieno[3,4-b]thiophene-6,4-diyl, and 3,6- pyrrolo[3,4-c]pyrrole-1,4-dione, all of which are optionally substituted by one or more, preferably one or two groups R,
wherein R is H, F, Cl or alkyl, alkoxy, thioalkyl, fluoroalkyl, carbonylalkyl, carbonyloxaalkyl, oxacarbonylalkyl, aryl, heteroaryl, aryloxy or heteroaryloxy with 1 to 30 C atoms, wherein the aryl and heteroaryl groups may also be substituted by fluoro, alkyl or alkoxy groups.

The n-type semiconductor can be an inorganic material such as zinc oxide (ZnOₓ), zinc tin oxide (ZTO), titanium oxide (TiOₓ), molybdenum oxide (MoOₓ), nickel oxide (NiOₓ), or cadmium selenide (CdSe), or an organic material such as graphene or a fullerene or substituted fullerene, for example an indene-C₆₀-fullerene bisaduct like ICBA, or a (6,6)-phenyl-butyric acid methyl ester derivatized methano C₆₀ fullerene, also known as "PCBM-C₆₀" or "C₆₀PCBM", as disclosed for example in G. Yu, J. Gao, J.C. Hummelen, F. Wudl, A.J. Heeger, Science 1995, Vol. 270, p. 1789 ff and having the structure shown below, or structural analogous compounds with e.g. a C₆₁ fullerene group, a C₇₀ fullerene group, or a C₇₁ fullerene group, or an organic polymer (see for example Coakley, K. M. and McGehee, M. D. Chem. Mater. 2004, 16, 4533).

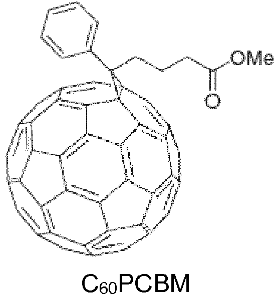

Preferably the p-type semiconductor is blended with an n-type semiconductor such as a fullerene or substituted fullerene, like for example PCBM-C₆₀, PCBM-C₇₀, PCBM-C₆₁, PCBM-C₇₁, bis-PCBM-C₆₁, bis-PCBM-C₇₁, ICBA (1',1",4',4"- tetrahydro-di[1,4]methanonaphthaleno [1,2:2',3';56,60:2",3"][5,6]fullerene-C60-Ih), graphene, or a metal oxide, like for example, ZnOₓ, TiOₓ, ZTO, MoOₓ, NiOₓ, to form the active layer in an OPV or OPD device.

The device preferably further comprises a first transparent or semi-transparent electrode on a transparent or semi-transparent substrate on one side of the active layer, and a second metallic or semi-transparent electrode on the other side of the active layer.

Further preferably the OPV or OPD device comprises, between the active layer and the first or second electrode, one or more additional buffer layers acting as hole transporting layer and/or electron blocking layer, which comprise a material such as metal oxide, like for example, ZTO, TiOₓ, MoOₓ, NiOₓ, a conjugated polymer electrolyte, like for example PEDOT:PSS, a conjugated polymer, like for example polytriarylamine (PTAA), an organic compound, like for example N,N'-diphenyl-N,N'-bis(1-naphthyl)(1,1'-biphenyl)-4,4'diamine (NPB), N,N'-diphenyl-N,N'-(3-methylphenyl)-1,1'-biphenyl-4,4'-diamine (TPD).

In the blend or mixture of a p-type semiconducting polymer and a fullerene or modified fullerene, the ratio polymer:fullerene is preferably from 5:1 to 1:5 by weight, more preferably from 1:1 to 1:3 by weight, most preferably 1:1 to 1:2 by weight. A polymeric binder may also be included, from 5 to 95% by weight. Examples of binder include polystyrene(PS), polypropylene (PP) and polymethylmethacrylate (PMMA).

To produce thin layers in BHJ OPV devices the compounds, polymers, polymer blends or formulations of the present invention may be deposited by any suitable method. Liquid coating of devices is more desirable than vacuum deposition techniques. Solution deposition methods are especially preferred. The formulations of the present invention enable the use of a number of liquid coating techniques. Preferred deposition techniques include, without limitation, dip coating, spin coating, ink jet printing, nozzle printing, letter-press printing, screen printing, gravure printing, doctor blade coating, roller printing, reverse-roller printing, offset lithography printing, dry offset lithography printing, flexographic printing, web printing, spray coating, dip coating, curtain coating, brush coating, slot dye coating or pad printing. For the fabrication of OPV devices and modules area printing method compatible with flexible substrates are preferred, for example slot dye coating, spray coating and the like.

Suitable solutions or formulations containing the blend or mixture of a p-type semiconducting polymer with a C₆₀ or C₇₀ fullerene or modified fullerene like PCBM must be prepared. In the preparation of formulations, suitable solvent must be selected to ensure full dissolution of both component, p-type and n-type and take into account the boundary conditions (for example rheological properties) introduced by the chosen printing method.

Organic solvents are generally used for this purpose. Typical solvents can be aromatic solvents, halogenated solvents or chlorinated solvents, including chlorinated aromatic solvents. Examples include, but are not limited to chlorobenzene, 1,2-dichlorobenzene, chloroform, 1,2-dichloroethane, dichloromethane, carbon tetrachloride, toluene, cyclohexanone, ethylacetate, tetrahydrofuran, anisole, morpholine, o-xylene, m-xylene, p-xylene, 1,4-dioxane, acetone, methylethylketone, 1,2-dichloroethane, 1,1,1-trichloroethane, 1,1,2,2-tetrachloroethane, ethyl acetate, n-butyl acetate, dimethylformamide, dimethylacetamide, dimethylsulfoxide, tetraline, decaline, indane, methyl benzoate, ethyl benzoate, mesitylene and combinations thereof.

In the OPV devices of the present invent invention the p-type and n-type semiconductor materials are preferably selected from the materials, like the polymer/fullerene systems, as described above
When the active layer is deposited on the substrate, it forms a BHJ that phase separate at nanoscale level. For discussion on nanoscale phase separation see Dennler et al, Proceedings of the IEEE, 2005, 93 (8), 1429 or Hoppe et al, Adv. Func. Mater, 2004, 14(10), 1005. An optional annealing step may be then necessary to optimize blend morpohology and consequently OPV device performance.

Another method to optimize device performance is to prepare formulations for the fabrication of OPV(BHJ) devices that may include high boiling point additives to promote phase separation in the right way. 1,8-octanedithiol, 1,8-diiodooctane, nitrobenzene, chloronaphthalene, and other additives have been used to obtain high-efficiency solar cells. Examples are disclosed in J. Peet, et al, Nat. Mater., 2007, 6, 497 or Fréchet et al. J. Am. Chem. Soc., 2010, 132, 7595-7597.

Alternatively, the present formulation can be used in a process for preparing an ETL in OLEDs. Common OLEDs are realized using multilayer structures. An emission layer is generally sandwiched between one or more electron-transport and/ or hole-transport layers. By applying an electric voltage electrons and holes as charge carriers move towards the emission layer where their recombination leads to the excitation and hence luminescence of the lumophor units contained in the emission layer. The formulations according to the present invention may be employed in one or more of the electron transport layers, The selection, characterization as well as the processing of the other layers and of the materials used therein is generally known by a person skilled in the art, see, e.g., Müller et al, Synth. Metals, 2000, 111-112, 31-34, Alcala, J. Appl. Phys., 2000, 88, 7124-7128 and the literature cited therein.

Unless the context clearly indicates otherwise, as used herein plural forms of the terms herein are to be construed as including the singular form and vice versa.

Throughout the description and claims of this specification, the words "comprise" and "contain" and variations of the words, for example "comprising" and "comprises", mean "including but not limited to", and are not intended to (and do not) exclude other components.

Above and below, unless stated otherwise percentages are per cent by weight and temperatures are given in degrees Celsius. The values of the dielectric constant ε ("permittivity") refer to values taken at 20°C and 1,000 Hz. Room temperature, unless stated otherwise, means 20°C.

It will be appreciated that variations to the foregoing embodiments of the invention can be made while still falling within the scope of the invention. Each feature disclosed in this specification, unless stated otherwise, may be replaced by alternative features serving the same, equivalent or similar purpose. Thus, unless stated otherwise, each feature disclosed is one example only of a generic series of equivalent or similar features.

All of the features disclosed in this specification may be combined in any combination, except combinations where at least some of such features and/or steps are mutually exclusive. In particular, the preferred features of the invention are applicable to all aspects of the invention and may be used in any combination. Likewise, features described in non-essential combinations may be used separately (not in combination).

The invention will now be described in more detail by reference to the following examples, which are illustrative only and do not limit the scope of the invention.

### Examples

### (Examples labelled "C" are comparison examples)

OPV devices having the following layer sequence were prepared as described below: Glass substrate / ITO electrode / HTL / BHJ / ETL / AI electrode
ITO patterned glass substrates are cleaned using ultra-sonic bath of successively: 3 min in acetone, 3min in IPA, 3min in deionized water. The HTL made of PEDOT:PSS is then deposited by spin-coating in air. The film is annealed at 130C during 30min under N₂. A volume of 80µL of the solution of BHJ is coated under N₂ using Doctor-Blade technique at a blade speed of 40mm/s, with a screw gap of 60µm, and maintaining the plate temperature at 70C. It is then dried 2min at 70C in N₂. A volume of 30µL of the ETL formulations are coated in air using a Doctor-Blade technique at a blade speed of 20mm/s, with a screw gap of 40µm, and maintaining the plate temperature at Room Temperature. Devices are then completed by a thermal evaporation of Aluminium in a special chamber having a pressure of 10⁻⁶mbar.

The photoactive layer with the BHJ comprises a blend of one of the conjugated polymers P1, P2, P3 and P4 as shown below, respectively, with the fullerene C₆₀-PCBM
P1: 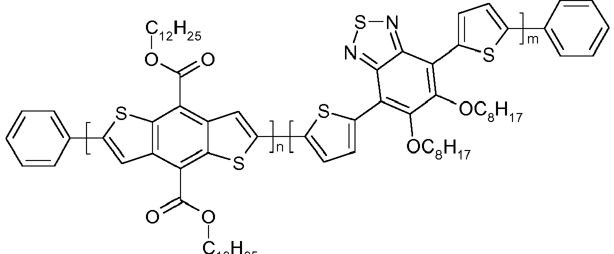 Mn = 27000g/mol, Mw = 65700g/mol, PDI = 2.41
P2: 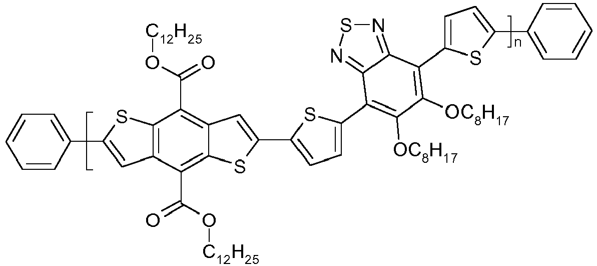 Mw = 41200g/mol, Mn = 22100g/mol, PDI = 1.86
P3: 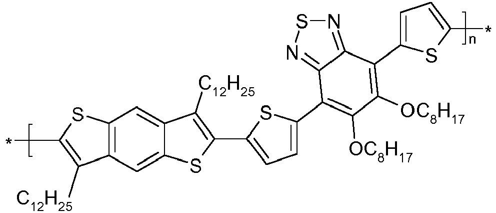 Mw = 60068g/mol, Mn = 39577g/mol, PDI = 1.52
P4: Poly(3-hexylthiophene), Mw = 77500g/mol , Mn = 38700g/mol

The ETL comprises a layer of a formulation comprising one of the salts S1, S2 and S3 or one of the ionic liquids IL1 to IL8 as shown below. Control devices were prepared having the same layer structure but without an ETL.
S1: 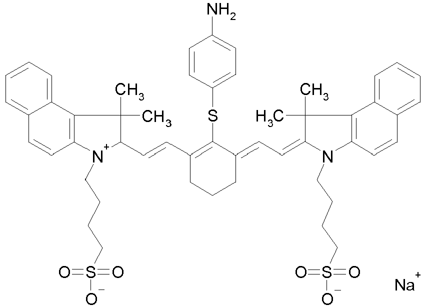
S2: 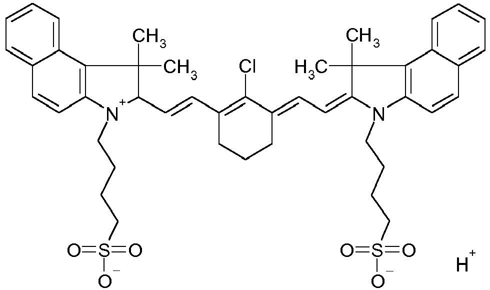
S3: 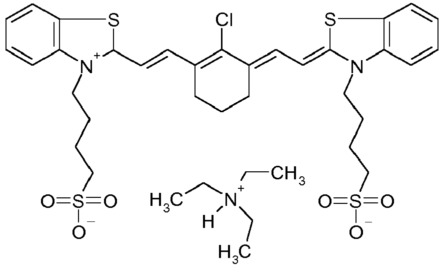
IL1 (not in accordance with the invention): 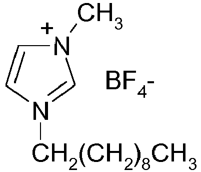
IL2 (not in accordance with the invention): 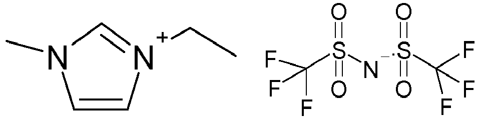
IL3 (not in accordance with the invention): 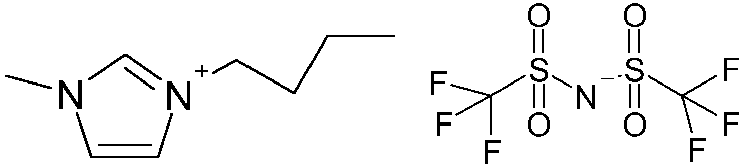
IL4: 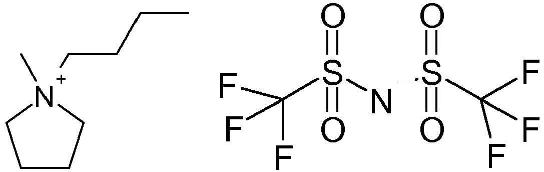
IL5: 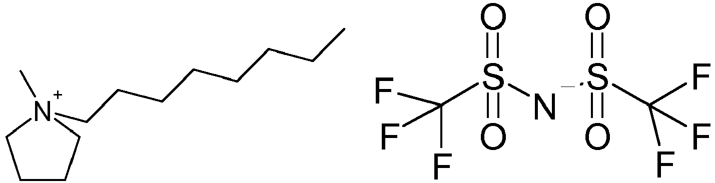
IL6: 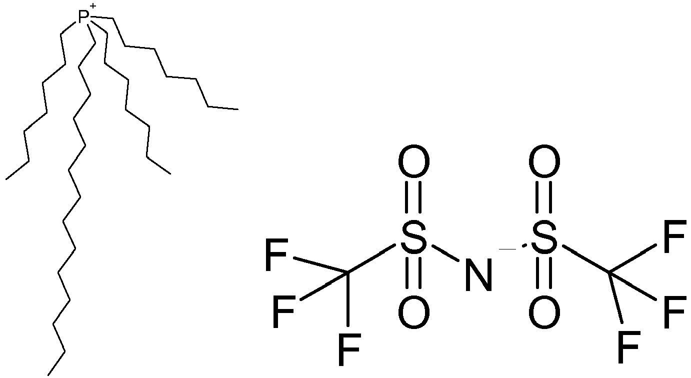
IL7 (not in accordance with the invention): 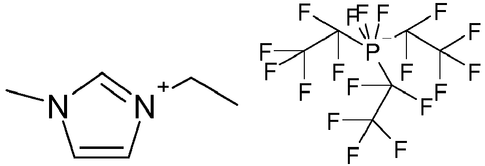
IL8 (not in accordance with the invention):

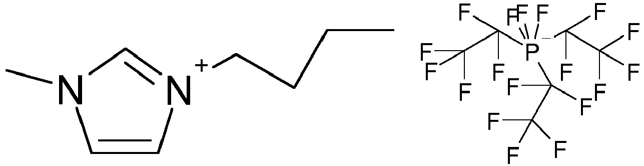

### Examples 1-6

The average parameters of the devices having the architecture {Glass/ITO/BHJ(Lisicon OPV polymer P1:PCBM)/ETL/AI} and using ETLs based on salt S1, S2 or S3, or without an ETL, are summarized in Table 1.

**Table 1**

| Ex. | ETL / cathode | | | Voc (mV) | Jsc (mA·cm²) | FF (%) | PCE (%) | PCE SD (%) | n_{ave/max} | Max. PCE (%) |
|---|---|---|---|---|---|---|---|---|---|---|
| | Salt | Cone. (mg/ml) | (H₂O:IPA) | | | | | | | |
| 1C | Al only | | | 743 | -10.91 | 69.4 | 5.63 | 0.11 | 0.98 | 5.77 |
| 2 | S1 | 0.6 | 4:1 | 806 | -11.01 | 74.5 | 6.60 | 0.18 | 0.97 | 6.80 |
| 3C | Al only | | | 751 | -11.10 | 70.2 | 5.85 | 0.10 | 0.97 | 6.02 |
| 4 | S2 | 0.6 | 4:1 | 800 | -11.06 | 73.6 | 6.51 | 0.22 | 0.94 | 6.94 |
| 5C | Al only | | | 751 | -11.10 | 70.2 | 5.85 | 0.10 | 0.97 | 6.02 |
| 6 | S3 | 0.6 | 1:4 | 770 | -11.42 | 71.3 | 6.27 | 0.24 | 0.96 | 6.54 |

Compared to the OPV devices of Examples 2, 4 and 6 in accordance with the invention, which comprise an ETL made from a formulation based on salt S1, S2 or S3, respectively, the reference devices of comparison examples 1C, 3C and 5C, without ETL, show lower performance.

### Examples 7-9

The average parameters of devices having the architecture {Glass/ITO/BHJ(Lisicon OPV Polymer P1:PCBM)/ETL/AI} and using an ETL based on salt S3 (Ex. 9), or without an ETL (Ex. 7C) or just washed by the solvent (Ex. 8C), are summarized in Table 2.

**Table 2**

| Ex. | ETL / cathode | | | V_{OC} (mV) | J_{SC} (mA·cm²) | FF (%) | PCE (%) | PCE SD (%) | n_{ave/max} | Max. PCE (%) |
|---|---|---|---|---|---|---|---|---|---|---|
| | Salt | Cone. (mg/ml) | (H₂O:IPA) | | | | | | | |
| 7C | Al only | | | 686 | -10.43 | 67.2 | 4.81 | 0.31 | 0.95 | 5.08 |
| 8C | Al only (BHJ is washed with the solvent used for depositing the formulation) | | | 647 | -9.34 | 65.8 | 3.98 | 0.37 | 0.91 | 4.37 |
| 9 | S3 | 1.25 | 4:1 | 800 | -10.74 | 72.6 | 6.24 | 0.33 | 0.95 | 6.59 |

Compared to the OPV device of Example 9 in accordance with the invention, which comprises an ETL made from a formulation based on salt S3, the reference devices of comparison examples 7C without ETL, or of Example 8C without ETL and wherein the BHJ is washed by the solvent used for depositing the formulation with S3 in Example 9, show lower performance.

### Examples 10-15

The average parameters of devices having the architecture {Glass/ITO/BHJ(Lisicon OPV Polymer P1:PCBM)/ETL/Al} and using ETLs based on salt 1 and different combinations of water and isopropanol, or without an ETL (Ex.10C), are summarized in Table 3.

**Table 3**

| Ex. | ETL / cathode | | V_{OC} (mV) | J_{SC} (mA·cm²) | FF (%) | PCE (%) | PCE SD (%) | n_{ave/max} | Max. PCE (%) |
|---|---|---|---|---|---|---|---|---|---|
| | Cone. of S3 (mg/ml) | (H₂O:IPA) | | | | | | | |
| 10C | Al only | | 729 | -6.22 | 65.6 | 2.97 | 0.15 | 0.93 | 3.19 |
| 11 | 1.25 | 4:1 | 793 | -8.84 | 66.9 | 4.68 | 0.40 | 0.91 | 5.15 |
| 12 | 1.25 | 2:1 | 787 | -9.00 | 69.2 | 4.90 | 0.33 | 0.94 | 5.19 |
| 13 | 1.25 | 1:1 | 750 | -8.86 | 54.6 | 3.62 | 0.56 | 0.90 | 4.02 |
| 14 | 1.25 | 1:2 | 784 | -8.96 | 66.7 | 4.69 | 0.42 | 0.92 | 5.08 |
| 15 | 1.25 | 1:4 | 740 | -8.06 | 64.8 | 3.87 | 0.59 | 0.83 | 4.64 |

Compared to the OPV devices of Examples 11-15 in accordance with the invention, which comprise an ETL made from a formulation based on salt S3 and various solvent combinations of water and isopropanol, respectively, the reference device of comparison examples 10C without ETL shows lower performance.

### Examples 16-22

The average parameters of devices having the architecture {Glass/ITO/BHJ (Lisicon OPV Polymer P1:PCBM)/ETL/AI} and using ETLs based on salt 1 in different concentrations, or without an ETL (Ex.16), are summarized in Table 4.

**Table 4**

| Ex. | ETL / cathode | | V_{OC} (mV) | J_{SC} (mA·cm²) | FF (%) | PCE (%) | PCE SD (%) | n_{ave/max} | Max. PCE (%) |
|---|---|---|---|---|---|---|---|---|---|
| | Cone. of S3 (mg/ml) | (H₂O:IPA) | | | | | | | |
| 16C | Al only | | 686 | -10.43 | 67.2 | 4.81 | 0.31 | 0.95 | 5.08 |
| 17 | 1.25 | 4:1 | 800 | -10.74 | 72.6 | 6.24 | 0.33 | 0.95 | 6.59 |
| 18 | 2.5 | 4:1 | 794 | -10.54 | 71.4 | 5.98 | 0.50 | 0.93 | 6.44 |
| 19 | 5.0 | 4:1 | 793 | -10.61 | 67.8 | 5.71 | 0.57 | 0.91 | 6.24 |
| 20C | Al only | | 735 | -10.44 | 65.5 | 5.03 | 0.24 | 0.97 | 5.19 |
| 21 | 1.25 | 4:1 | 812 | -10.67 | 73.3 | 6.34 | 0.14 | 0.97 | 6.56 |
| 22 | 0.6 | 4:1 | 808 | -10.46 | 73.7 | 6.24 | 0.40 | 0.93 | 6.71 |

Compared to the OPV devices of Examples 17-19 and 21-22 in accordance with the invention, which comprise an ETL made from a formulation based on salt S3 in various concentrations, respectively, the reference devices of comparison examples 16C and 20C, without ETL, show lower performance.

### Examples 23-25

The average parameters of devices having the architecture {Glass/ITO/BHJ(Lisicon OPV Polymer P1:PCBM)/ETL/AI} and using ETLs based on salt S1 (Ex. 25) or calcium (Ex.23C), or without an ETL (Ex.24C), are summarized in Table 5.

**Table 5**

| Ex. | ETL / cathode | V_{OC} (mV) | J_{SC} (mA·cm²) | FF (%) | PCE (%) | PCE SD (%) | n_{ave/max} | Max. PCE(%) |
|---|---|---|---|---|---|---|---|---|
| 23C | Ca / Al | 807 | -9.92 | 76.0 | 6.08 | 0.36 | 0.95 | 6.42 |
| 24C | Al only | 735 | -10.44 | 65.5 | 5.03 | 0.24 | 0.97 | 5.19 |
| 25 | S3 / Al | 812 | -10.67 | 73.3 | 6.34 | 0.14 | 0.97 | 6.56 |

Compared to the OPV devices of Example 25 in accordance with the invention, which comprises an ETL made from a formulation based on salt S1, the reference devices of comparison examples 23C and 24C, with a calcium ETL or without ETL, respectively show lower performance.

### Examples 26-33

The average parameters of devices having the architecture {Glass/ITO/BHJ/ETL/Al} and using ETLs based on salt 1 and different electron-donating polymers in the BHJs are summarized in Table 6.

**Table 6**

| Ex. | BHJ | ETL/ cathode | V_{OC} (mV) | J_{SC} (mA·cm²) | FF (%) | PCE (%) | PCE SD (%) | n_{ave/max} | Max. PCE (%) |
|---|---|---|---|---|---|---|---|---|---|
| 26C | P1:PCBM | Al only | 721 | -10.42 | 64.2 | 4.82 | 0.19 | 0.95 | 5.09 |
| 27 | P1:PCBM | S1 / Al | 807 | -10.82 | 71.3 | 6.22 | 0.30 | 0.93 | 6.68 |
| 28C | P4:PCBM | Al only | 327 | -5.41 | 51.8 | 0.92 | 0.05 | 0.94 | 0.98 |
| 29 | P4:PCBM | S1 / Al | 481 | -5.54 | 54.4 | 1.45 | 0.25 | 0.77 | 1.89 |
| 30C | P2:PCBM | Al only | 621 | -7.92 | 42.7 | 2.11 | 0.32 | 0.90 | 2.33 |
| 31 | P2:PCBM | S1 / Al | 619 | -8.35 | 43.4 | 2.25 | 0.17 | 0.91 | 2.46 |
| 32C | P3:PCBM | Al only | 803 | -6.51 | 38.0 | 1.99 | 0.06 | 0.94 | 2.11 |
| 33 | P3:PCBM | S1 / Al | 948 | -6.97 | 43.2 | 2.85 | 0.11 | 0.95 | 3.01 |

Compared to the OPV devices of Examples 27, 29, 31 and 33 in accordance with the invention, which comprise an ETL made from a formulation based on salt S1, respectively, the reference devices of comparison examples 28C, 30C and 32C, without ETL, show lower performance, even if the composition of the BHJ is changed.

### Examples 34-35 (not in accordance with the invention)

The average parameters of devices having the architecture {Glass/ITO/BHJ(Lisicon OPV Polymer P1 :PCBM)/ETL/AI} and using an ETL obtained from a formulation comprising 0.5 vol% ionic liquid IL1 and 99.5 vol% methanol (Ex. 35), or without an ETL (Ex. 34C), are summarized in Table 7.

**Table 7 (not in accordance with the invention)**

| Ex. | ETL / cathode | | | V_{OC} (mV) | J_{SC} (mA·cm²) | FF (%) | PCE (%) | PCE SD (%) | n_{ave/max} | Max. PCE (%) |
|---|---|---|---|---|---|---|---|---|---|---|
| | IL Type | Speed (rpm) | (vol% IL) | | | | | | | |
| 34C | Al only | | | 729 | -13.60 | 64.2 | 6.36 | 0.31 | 0.91 | 6.95 |
| 35 | IL1 | 2000 | 0.5 | 752 | -13.53 | 65.7 | 6.68 | 0.22 | 0.94 | 7.08 |

Compared to the OPV device of Example 35 in accordance with the invention, which comprises an ETL made from a formulation based on ionic liquid IL1 and methanol, the reference device of comparison example 34C without ETL shows lower performance.

### Examples 36-37 (not in accordance with the invention)

The average parameters of devices having the architecture {Glass/ITO/BHJ(Lisicon OPV Polymer P1 :PCBM)/ETL/AI} and using an ETL obtained from a formulation comprising 0.018 vol% ionic liquid IL1 and 99.982 vol% isopropanol (Ex. 37), or without an ETL (Ex. 36C), are summarized in Table 8.

**Table 8 (not in accordance with the invention)**

| Ex. | ETL / cathode | | V_{OC} (mV) | J_{SC} (mA·cm²) | FF (%) | PCE (%) | PCE SD (%) | n_{ave/max} | Max. PCE (%) |
|---|---|---|---|---|---|---|---|---|---|
| | IL Type | (vol% IL) | | | | | | | |
| 36C | Al only | | 736 | -11.41 | 68.9 | 5.79 | 0.58 | 0.84 | 6.35 |
| 37 | IL1 | 0.018 | 795 | -12.10 | 72.1 | 6.93 | 0.23 | 0.95 | 7.26 |

Compared to the OPV device of Example 37 in accordance with the invention, which comprises an ETL made from a formulation based on ionic liquid IL1 and isopropanol, the reference device of comparison example 36C without ETL shows lower performance.

### Examples 38-41 (not in accordance with the invention)

The average parameters of devices having the architecture {Glass/ITO/BHJ(Lisicon OPV Polymer P1 :PCBM)/ETL/AI} and using an ETL obtained from a formulation comprising ionic liquid IL1 and isopropanol in different volumic ratios (Ex. 39-41), or without an ETL (Ex. 38C), are summarized in Table 9.

**Table 9 (not in accordance with the invention)**

| Ex. | ETL / cathode | | V_{OC} (mV) | J_{SC} (mA·cm²) | FF (%) | PCE (%) | PCE SD (%) | n_{ave/max} | Max. PCE (%) |
|---|---|---|---|---|---|---|---|---|---|
| | IL Type | (vol% IL) | | | | | | | |
| 38C | Al only | | 736 | -11.41 | 68.9 | 5.79 | 0.58 | 0.84 | 6.35 |
| 39 | IL1 | 0.036 | 793 | -11.38 | 69.8 | 6.30 | 0.15 | 0.96 | 6.55 |
| 40 | IL1 | 0.018 | 795 | -12.10 | 72.1 | 6.93 | 0.23 | 0.95 | 7.26 |
| 41 | IL1 | 0.0036 | 800 | -12.18 | 71.4 | 6.96 | 0.18 | 0.97 | 7.20 |

Compared to the OPV devices of Examples 39-41 in accordance with the invention, which comprises an ETL made from a formulation based on ionic liquid IL1 and isopropanol in different ratios, the reference device of comparison example 38C without ETL shows lower performance. Table 9 also shows that the varying the concentration of the IL in the solvent the performance can be optimised.

### Examples 42-44 (not in accordance with the invention)

The average parameters of devices having the architecture {Glass/ITO/BHJ(Lisicon OPV Polymer P1 :PCBM)/ETL/AI} and using an ETL obtained from a formulation comprising 0.18 vol% ionic liquid IL1 and 99.82 vol% of either isopropanol or methanol (Ex. 43-44), or without an ETL (Ex. 42C), are summarized in Table 10.

**Table 10 (not in accordance with the invention)**

| Ex. | ETL / cathode | | V_{OC} (mV) | J_{SC} (mA·cm²) | FF (%) | PCE (%) | PCE SD (%) | n_{ave/max} | Max. PCE (%) |
|---|---|---|---|---|---|---|---|---|---|
| | IL Type | (vol% IL) | | | | | | | |
| 42C | Al only | | 736 | -11.41 | 68.9 | 5.79 | 0.58 | 0.84 | 6.35 |
| 43 | IL1 | 0.18 methanol | 738 | -11.19 | 52.5 | 4.34 | 0.56 | 0.83 | 5.23 |
| 44 | IL1 | 0.18 isopropanol | 773 | -11.97 | 69.1 | 6.39 | 0.26 | 0.95 | 6.74 |

The OPV device of Example 44 in accordance with the invention, which comprises an ETL made from a formulation based on ionic liquid IL1 and isopropanol shows better performance than the reference device of comparison example 42C without ETL.

On the other hand, the OPV device of Example 43 in accordance with the invention, which comprises an ETL made from a formulation based on ionic liquid IL1 and methanol shows lower performance, and also lower performance than the device of example 35 with a higher ratio of IL1 in methanol. This shows that by varying the concentration of the IL in the solvent the performance can be optimised.

### Examples 45-46

The average parameters of devices having the architecture {Glass/ITO/BHJ(Lisicon OPV Polymer P1:PCBM)/ETL/Ag/Al} and using ETLs based on salt S1 (Ex. 46) or without an ETL (Ex.45C), are summarized in Table 11.

**Table 11**

| Ex. | ETL / cathode | V_{OC} (mV) | J_{SC} (mA·cm²) | FF (%) | PCE (%) | Max. PCE(%) |
|---|---|---|---|---|---|---|
| 45C | Ag / Al | 754 | 12.48 | 67.6 | 6.36 | 6.96 |
| 46 | S1 | 800 | 14.58 | 70.8 | 8.27 | 9.09 |

Compared to the OPV devices of Example 46C in accordance with the invention, which comprise an ETL made from a formulation based on salt S1, S2 or S3, respectively, the reference devices of comparison example 45 without ETL, show lower performance.

### Examples 47-56

The average parameters of devices having the architecture {Glass/ITO/BHJ(Lisicon OPV Polymer P1:PCBM)/ETL/Ag/Al} and using ETLs based on ionic liquid IL2, or ionic liquid IL3, or ionic liquid IL3, or ionic liquid IL4, or ionic liquid IL5, or ionic liquid IL6, or ionic liquid IL7, or ionic liquid IL8 or without an ETL (Ex.47C), are summarized in Table 12.

**Table 12**

| Ex. | ETL / cathode | V_{OC} (mV) | J_{SC} (mA·cm²) | FF (%) | PCE (%) |
|---|---|---|---|---|---|
| 47C | Ag / Al | 754 | 12.48 | 67.6 | 6.36 |
| 48 (not in accordance with the invention) | IL2 | 797 | 13.4 | 71.2 | 7.62 |
| 49 (not in accordance with the invention) | IL3 | 774 | 13.6 | 68.6 | 7.21 |
| 50 | IL4 | 790 | 13.3 | 71.2 | 7.50 |
| 51 | IL5 | 801 | 12.66 | 70.1 | 7.11 |
| 52 | IL6 | 801 | 13.1 | 70.2 | 7.38 |
| 53 (not in accordance with the invention) | IL7 | 801 | 12.9 | 71.6 | 7.39 |
| 54 (not in accordance with the invention) | IL8 | 798 | 13.9 | 67.6 | 7.49 |

Compared to the OPV devices of Examples 48 (not in accordance with the invention), 49 (not in accordance with the invention), 50, 51, 52, 53 (not in accordance with the invention), 54 (not in accordance with the invention), which comprise an ETL made from a formulation based on an ionic liquid as IL2 (not in accordance with the invention), or IL3 (not in accordance with the invention), or IL4, or IL5, or IL6, or IL7 (not in accordance with the invention), or IL8 (not in accordance with the invention), respectively, the reference devices of comparison example 47C without ETL, show lower performance.

## Claims

1. Device selected from organic photovoltaic devices (OPV), organic solar cells, photodetectors, or a component thereof, or an assembly comprising it, which comprises a layer comprising an organic salt, wherein the organic salt comprises a first ionic entity and a second counterionic entity, wherein said layer is an electron transport layer (ETL) in the device and wherein
- the first and second entities are small molecules, and
- the first entity is an organic entity, comprising
- a charged organic core comprising a single charged moiety or a plurality of charged moieties, and
- optionally one or more substituents attached to the charged organic core, at least one of said substituents comprising a charged moiety having a charge that is opposite in sign to, or of the same sign as, the charged moieties of the charged organic core, and
- the second entity is an organic, inorganic or organometallic entity, and
- the net charge of the first entity is opposite in sign to the net charge of the second entity,
**characterised in that** the first entity comprises a moiety selected from the group consisting of dialkylpyrrolidinium, trialkylsulfonium, carbazolium, indolium, piperidinium, morpholinium, pyrimidinium, pyridazinium, pyrazinium, pyrazolium, pyrrolinium, pyrimidinium, pyrazinium, thiazolium, oxazolium, triazolium, triazinium, guanidinium, uranium, sulfonium, phosphonium.

2. Device according to claim 1, wherein the second entity comprises a halide, borate, imide, nitrate, phosphate, sulfonate, sulfate, succinate, naphthenate, carboxylate or O-heterocyclic moiety.

3. Device according to claim 1 or 2, wherein the second entity comprises a moiety selected from the group consisting of imidazolium, dialkylimidazolium, trialkylimidazolium, dialkylpyrrolidinium, mono or dialkylpyridinium, trialkylsulfonium, carbazolium, indolium, piperidinium, morpholinium, pyrimidinium, pyridazinium, pyrazinium, pyrazolium, pyrrolinium, pyrimidinium, pyrazinium, thiazolium, oxazolium, triazolium, triazinium, guanidinium, uranium, sulfonium, phosphonium.

4. Device according to one or more of claims 1 to 3, wherein the first entity comprises an organic zwitterionic compound, and the second entity comprises an organic or inorganic counterion.

5. Device according to claim 4, wherein the first entity comprises a zwitterionic dye, preferably selected from a cyanine dye, a merocyanine dye, a squarylium dye, or a polymethine dye.

6. Device according to one or more of claims 1 to 5, wherein the organic salt is an ionic liquid (IL) or a polymerisable ionic liquid (PIL).

7. A process of preparing the electron transport layer as defined in claim 1, comprising the steps of depositing a formulation, which comprises an organic salt as defined in one or more of claims 1 to 6 and one or more solvents on a substrate and removing the solvents.

8. Process according to claim 7, wherein the solvents are selected from water, alcohols and polar non-alcoholic organic solvents, or combinations of the aforementioned.

9. Process according to claim 8, wherein the solvent is selected from isopropanol, ethanol, a combination of water and isopropanol, or a combination of water and ethanol.

10. Process according to one or more of claims 7 to 9, wherein the concentration of the salt in the solvents is from 0.001mg/ml to 30mg/ml.

11. Process according to one or more of claims 7 to 10, wherein the organic salt is an ionic liquid (IL) or a polymerisable ionic liquid (PIL) and the concentration of the salt in the solvents is from 0.0001 to 10 vol%.

12. The process according to one or more of claims 7 to 11, wherein the formulation is deposited by spin-coating or printing, preferably slot-dye coating, inkjet printing, spraying or doctor-blading.

13. The process according to one or more of claims 7 to 12, wherein the formulation is processed under an atmosphere of air, N₂ or water pressure.

14. The process according to one or more of claims 7 to 13, wherein the formulation is deposited at a temperature from 10°C to 120°C, preferably at room temperature.

15. The process according to one or more of claims 7 to 14, wherein the layer is dried at a temperature from room temperature to 200°C, preferably from room temperature to 60°C.

16. The process according to one or more of claims 7 to 15, wherein the layer is formed on a substrate which is selected from an electrode, an ETL, a HTL, a BHJ, a plastic substrate, a glass substrate, a conducting layer or a semiconducting layer, preferably selected from a BHJ in an OPV device or a metal oxide semiconducting layer, conducting layer or ETLs in an inverted OPV device.

17. The device according to any one of claims 1 to 6, which is a bulk heterojunction (BHJ) OPV device or inverted BHJ OPV device.

18. The device according to claim 17, which is a BHJ OPV device or an inverted BHJ OPV device comprising a cathode of aluminium, gold, silver, calcium, magnesium or barium.

19. The component according to any one of claims 1 to 6, which is selected from semiconductor films, charge injection layers, charge transport layers, interlayers, planarising layers, antistatic films, polymer electrolyte membranes (PEM), laser materials, conducting substrates and conducting patterns.

## Patentansprüche

1. Vorrichtung ausgewählt aus organischen Photovoltaikvorrichtungen (OPV), organischen Solarzellen, Photodetektoren oder einem Bauteil davon oder einer dieses enthaltenden Baugruppe, die eine Schicht mit einem organischen Salz enthält, wobei das organische Salz eine erste ionische Einheit und eine zweite gegenionische Einheit enthält, wobei die Schicht eine Elektronentransportschicht (electron transport layer - ETL) in der Vorrichtung ist und wobei
- die ersten und zweiten Einheiten kleine Moleküle sind, und
- die erste Einheit eine organische Einheit ist, enthaltend
- einen geladenen organischen Kern, der einen einzelnen geladenen Molekülteil oder eine Vielzahl von geladenen Molekülteilen enthält, und
- gegebenenfalls einen oder mehrere an den geladenen organischen Kern gebundene Substituenten, wo mindestens einer der Substituenten einen geladenen Molekülteil mit einer Ladung enthält, deren Vorzeichen dem der geladenen Molekülteile des geladenen organischen Kerns entgegengesetzt oder ihm gleich ist, und
- die zweite Einheit eine organische, anorganische oder metallorganische Einheit ist, und
- die Nettoladung der ersten Einheit im Zeichen entgegengesetzt zur Nettoladung der zweiten Einheit ist,
**dadurch gekennzeichnet, dass** die erste Einheit einen Molekülteil enthält, der aus der Gruppe bestehend aus Dialkylpyrrolidinium, Trialkylsulfonium, Carbazolium, Indolium, Piperidinium, Morpholinium, Pyrimidinium, Pyridazinium, Pyrazinium, Pyrazolium, Pyrrolinium, Thiazolium, Oxazolium, Triazolium, Triazinium, Guanidinium, Uranium, Sulfonium, Phosphonium ausgewählt ist.

2. Vorrichtung nach Anspruch 1, wobei die zweite Einheit ein Halogenid, Borat, Imid, Nitrat, Phosphat, Sulfonat, Sulfat, Succinat, Naphthenat, Carboxylat oder einen O-heterocyclischen Molekülteil enthält.

3. Vorrichtung nach Anspruch 1 oder 2, wobei die zweite Einheit einen Molekülteil enthält, der aus der Gruppe bestehend aus Imidazolium, Dialkylimidazolium, Trialkylimidazolium, Dialkylpyrrolidinium, Mono- oder Dialkylpyridinium, Trialkylsulfonium, Carbazolium, Indolium, Piperidinium, Morpholinium, Pyrimidinium, Pyridazinium, Pyrazinium, Pyrazolium, Pyrrolinium, Thiazolium, Oxazolium, Triazolium, Triazinium, Guanidinium, Uranium, Sulfonium, Phosphonium ausgewählt ist.

4. Vorrichtung nach einem oder mehreren der Ansprüche 1 bis 3, wobei die erste Einheit eine organische zwitterionische Verbindung enthält und die zweite Einheit ein organisches oder anorganisches Gegenion enthält.

5. Vorrichtung nach Anspruch 4, wobei die erste Einheit einen zwitterionischen Farbstoff enthält, der vorzugsweise aus einem Cyaninfarbstoff, einem Merocyaninfarbstoff, einem Squaryliumfarbstoff oder einem Polymethinfarbstoff ausgewählt ist.

6. Vorrichtung nach einem oder mehreren der Ansprüche 1 bis 5, wobei es sich bei dem organischen Salz um eine ionische Flüssigkeit (IL) oder eine polymerisierbare ionische Flüssigkeit (PIL) handelt.

7. Verfahren zur Herstellung der Elektronentransportschicht wie in Anspruch 1 definiert, umfassend die Schritte Abscheiden einer Formulierung, die ein organisches Salz wie in einem oder mehreren der Ansprüche 1 bis 6 definiert und ein oder mehrere Lösungsmittel enthält, auf einem Substrat und Entfernen der Lösungsmittel.

8. Verfahren nach Anspruch 7, wobei die Lösungsmittel aus Wasser, Alkoholen und polaren nicht-alkoholischen organischen Lösungsmitteln oder Kombinationen der vorgenannten ausgewählt sind.

9. Verfahren nach Anspruch 8, wobei das Lösungsmittel aus Isopropanol, Ethanol, einer Kombination aus Wasser und Isopropanol oder einer Kombination aus Wasser und Ethanol ausgewählt ist.

10. Verfahren nach einem oder mehreren der Ansprüche 7 bis 9, wobei die Konzentration des Salzes in den Lösungsmitteln 0,001mg/ml bis 30mg/ml beträgt.

11. Verfahren nach einem oder mehreren der Ansprüche 7 bis 10, wobei es sich bei dem organischen Salz um eine ionische Flüssigkeit (IL) oder eine polymerisierbare ionische Flüssigkeit (PIL) handelt und die Konzentration des Salzes in den Lösungsmitteln 0,0001 bis 10 Vol.-% beträgt.

12. Verfahren nach einem oder mehreren der Ansprüche 7 bis 11, wobei die Formulierung durch Schleuderbeschichten oder Drucken, vorzugsweise Schlitzdüsenbeschichtung, Tintenstrahldruck, Sprühen oder Rakeln abgeschieden wird.

13. Verfahren nach einem oder mehreren der Ansprüche 7 bis 12, wobei die Formulierung unter einer Atmosphäre von Luft, N₂ oder Wasserdruck verarbeitet wird.

14. Verfahren nach einem oder mehreren der Ansprüche 7 bis 13, wobei die Formulierung bei einer Temperatur von 10°C bis 120°C, vorzugsweise bei Raumtemperatur abgeschieden wird.

15. Verfahren nach einem oder mehreren der Ansprüche 7 bis 14, wobei die Schicht bei einer Temperatur von Raumtemperatur bis 200°C, vorzugsweise von Raumtemperatur bis 60°C getrocknet wird.

16. Verfahren nach einem oder mehreren der Ansprüche 7 bis 15, wobei die Schicht auf einem Substrat gebildet wird, das aus einer Elektrode, einer ETL, einer HTL, einem BHJ, einem Kunststoffsubstrat, einem Glassubstrat, einer Leiterschicht oder einer Halbleiterschicht ausgewählt ist, vorzugsweise aus einem BHJ in einer OPV-Vorrichtung oder einer Metalloxid-Halbleiterschicht, Leiterschicht oder ETLs in einer invertierten OPV-Vorrichtung ausgewählt ist.

17. Vorrichtung nach einem beliebigen der Ansprüche 1 bis 6, bei der es sich um eine OPV-Vorrichtung mit Volumen-Heteroübergang (bulk heterojunction - BHJ) oder invertierte BHJ-OPV-Vorrichtung handelt.

18. Vorrichtung nach Anspruch 17, bei der es sich um eine BHJ-OPV-Vorrichtung oder eine invertierte BHJ-OPV-Vorrichtung mit einer Kathode aus Aluminium, Gold, Silber, Calcium, Magnesium oder Barium handelt.

19. Bauteil nach einem beliebigen der Ansprüche 1 bis 6, das aus Halbleiterfolien, Ladungsinjektionsschichten, Ladungstransportschichten, Zwischenschichten, Planarisierungsschichten, Antistatikfolien, Polymerelektrolytmembranen (PEM), Lasermaterialien, leitenden Substraten und leitenden Strukturen ausgewählt ist.

## Revendications

1. Dispositif sélectionné parmi les dispositifs photovoltaïques organiques (OPV), les cellules solaires organiques, les photodétecteurs, ou un composant de ceux-ci, ou un assemblage le comprenant, lequel comprend une couche qui comprend un sel organique, dans lequel le sel organique comprend une première entité ionique et une seconde entité contre ionique, dans lequel ladite couche est une couche de transport d'électrons (ETL) dans le dispositif et dans lequel :
- les première et seconde entités sont des petites molécules, et
- la première entité est une entité organique, comprenant :
- un cœur organique chargé qui comprend une unique moitié chargée ou une pluralité de moitiés chargées, et
- en option, un ou plusieurs substituant(s) qui est/sont lié(s) au cœur organique chargé, au moins l'un desdits substituants comprenant une moitié chargée qui comporte une charge qui est opposée en termes de signe au signe des moitiés chargées du cœur organique chargé ou qui est du même signe que le signe de ces mêmes moitiés, et
- la seconde entité est une entité organique, inorganique ou organométallique, et
- la charge nette de la première entité est opposée en termes de signe à la charge nette de la seconde entité,
**caractérisé en ce que** :
la première entité comprend une moitié qui est sélectionnée parmi le groupe qui est constitué par le dialkylpyrrolidinium, le trialkylsulfonium, le carbazolium, l'indolium, le pipéridinium, le morpholinium, le pyrimidinium, le pyridazinium, le pyrazinium, le pyrazolium, le pyrrolinium, le thiazolium, l'oxazolium, le triazolium, le triazinium, le guanidinium, l'uranium, le sulfonium et le phosphonium.

2. Dispositif selon la revendication 1, dans lequel la seconde entité comprend une moitié halogénure, borate, imide, nitrate, phosphate, sulfonate, sulfate, succinate, naphténate, carboxylate ou O-hétérocyclique.

3. Dispositif selon la revendication 1 ou 2, dans lequel la seconde entité comprend une moitié qui est sélectionnée parmi le groupe qui est constitué par l'imidazolium, le dialkylimidazolium, le trialkylimidazolium, le dialkylpyrrolidinium, le mono ou dialkylpyridinium, le trialkylsulfonium, le carbazolium, l'indolium, le pipéridinium, le morpholinium, le pyrimidinium, le pyridazinium, le pyrazinium, le pyrazolium, le pyrrolinium, le pyrimidinium, le pyrazinium, le thiazolium, l'oxazolium, le triazolium, le triazinium, le guanidinium, l'uranium, le sulfonium et le phosphonium.

4. Dispositif selon une ou plusieurs des revendications 1 à 3, dans lequel la première entité comprend un composé zwitterionique organique et la seconde entité comprend un contre-ion organique ou inorganique.

5. Dispositif selon la revendication 4, dans lequel la première entité comprend un colorant zwitterionique, de préférence sélectionné parmi un colorant cyanine, un colorant mérocyanine, un colorant squarylium et un colorant polyméthine.

6. Dispositif selon une ou plusieurs des revendications 1 à 5, dans lequel le sel organique est un liquide ionique (IL) ou un liquide ionique polymérisable (PIL).

7. Procédé de préparation de la couche de transport d'électrons telle que définie selon la revendication 1, comprenant les étapes de dépôt d'une formulation, qui comprend un sel organique tel que défini selon une ou plusieurs des revendications 1 à 6 et un ou plusieurs solvant(s) sur un substrat, et d'enlèvement des solvants.

8. Procédé selon la revendication 7, dans lequel les solvants sont sélectionnés parmi l'eau, les alcools et les solvants organiques non alcooliques polaires ou des combinaisons des solvants qui ont été mentionnés.

9. Procédé selon la revendication 8, dans lequel le solvant est sélectionné parmi l'isopropanol, l'éthanol, une combinaison d'eau et d'iso-propanol, ou une combinaison d'eau et d'éthanol.

10. Procédé selon une ou plusieurs des revendications 7 à 9, dans lequel la concentration du sel dans les solvants va de 0,001 mg/ml à 30 mg/ml.

11. Procédé selon une ou plusieurs des revendications 7 à 10, dans lequel le sel organique est un liquide ionique (IL) ou un liquide ionique polymérisable (PIL) et la concentration du sel dans les solvants va de 0,0001 à 10 % en volume.

12. Procédé selon une ou plusieurs des revendications 7 à 11, dans lequel la formulation est déposée au moyen d'un revêtement par centrifugation ou d'une impression, de préférence au moyen d'un revêtement par filière plate, d'une impression par jet d'encre, d'une pulvérisation ou d'un revêtement par lame docteur.

13. Procédé selon une ou plusieurs des revendications 7 à 12, dans lequel la formulation est traitée sous une atmosphère de pression d'air, de N₂ ou d'eau.

14. Procédé selon une ou plusieurs des revendications 7 à 13, dans lequel la formulation est déposée à une température qui va de 10 °C à 120 °C, de préférence à température ambiante.

15. Procédé selon une ou plusieurs des revendications 7 à 14, dans lequel la couche est séchée à une température qui va de la température ambiante à 200 °C, de préférence de la température ambiante à 60 °C.

16. Procédé selon une ou plusieurs des revendications 7 à 15, dans lequel la couche est formée sur un substrat qui est sélectionné parmi une électrode, une ETL, une HTL, une BHJ, un substrat en matière plastique, un substrat en verre, une couche de conduction ou une couche de semiconduction, le substrat étant de préférence sélectionné parmi une BHJ dans un dispositif OPV ou une couche de semiconduction en oxyde métallique, une couche de conduction ou des ETL dans un dispositif OPV inversé.

17. Dispositif selon l'une quelconque des revendications 1 à 6, lequel est un dispositif OPV à hétérojonction dans la masse (BHJ) ou un dispositif OPV BHJ inversé.

18. Dispositif selon la revendication 17, lequel est un dispositif OPV BHJ ou un dispositif OPV BHJ inversé qui comprend une cathode en aluminium, en or, en argent, en calcium, en magnésium ou en baryum.

19. Composant selon l'une quelconque des revendications 1 à 6, lequel est sélectionné parmi les films semiconducteurs, les couches d'injection de charges, les couches de transport de charges, les inter-couches, les couches de planarisation, les films antistatiques, les membranes électrolytiques polymère (PEM), les matériaux laser, les substrats de conduction et les motifs de conduction.
